# EUROPEAN PATENT SPECIFICATION

(11) **EP 3 919 093 B1**
(45) Date of publication and mention of the grant of the patent: **19.11.2025**
(21) Application number: 20762342.2
(22) Date of filing: 20.02.2020
(51) Int. Cl.: A61L 29/08, A61L 29/14, B05D 5/00, B05D 5/08

(54) **MEDICAL INSTRUMENT MANUFACTURING METHOD AND MEDICAL INSTRUMENT**
VERFAHREN ZUR HERSTELLUNG EINES MEDIZINISCHEN INSTRUMENTS SOWIE MEDIZINISCHES INSTRUMENT
PROCÉDÉ DE FABRICATION D'INSTRUMENT MÉDICAL ET INSTRUMENT MÉDICAL

(30) Priority: 27.02.2019 JP 2019034808
(43) Date of publication of application: 08.12.2021
(73) Proprietor: TERUMO Kabushiki Kaisha, Tokyo 151-0072 (JP)
(72) Inventor: KOHAMA, Hiromasa, Ashigarakami-gun, Kanagawa 259-0151 (JP); KONDO, Keiko, Ashigarakami-gun, Kanagawa 259-0151 (JP)
(74) Representative: Casalonga
(86) International application number: PCT/JP2020/006890
(87) International publication number: WO 2020/175329

(56) References cited:
- EP-A1- 2 380 600
- EP-A1- 3 225 260
- WO-A1-2013/105394
- WO-A1-2016/084716
- JP-A- 2002 249 707
- JP-A- H0 824 327
- JP-A- H11 116 667

## Description

### Technical Field

The present invention relates to a method for manufacturing a medical instrument, and a medical instrument.

### Background Art

Medical instruments that are inserted into a living body, such as catheters and guide wires, are required to exhibit an excellent lubricating property in order to reduce damage to tissue such as blood vessels and to enhance the maneuverability of the operator. Therefore, methods of coating the surface of a base layer of a medical instrument with a hydrophilic polymer having a lubricating property, have been developed and put into practical use. On the other hand, it is also important that such a medical instrument can maintain the hydrophilic polymer having a lubricating property on the surface of the base layer when the operator uses the medical instrument, in order to maintain the maneuverability of the operator. Therefore, a coating formed with a hydrophilic polymer is required to have not only an excellent lubricating property but also durability against loads such as abrasion and scratching.

From such a viewpoint, JP-A-8-24327 describes a medical instrument having a lubricating layer on a substrate. The lubricating layer is formed from a hydrophilic polymer having a reactive functional group, such as an epoxy group, and a hydrophilic monomer, a polymer having a functional group capable of reacting with the reactive functional group of the hydrophilic polymer, and a solvent.

Another document, JP-A-8-33704, discloses a medical instrument having a surface lubricating layer formed on the surface of a base layer, which is obtained by dissolving a water-soluble or water-swellable polymer in a solvent in which a base of a medical instrument swells to produce a polymer solution, immersing the base of the medical instrument in this polymer solution to swell, and cross-linking or polymerizing this polymer at the surface of the base layer.

According to the technology disclosed in JP-A-8-33704, a surface lubricating layer exhibiting a favorable lubricating property can be fixed to a base.

### Summary of Invention

### Technical Problem

In JP-A-8-33704, it is disclosed that it is preferable to use a block copolymer comprising a hydrophilic moiety exhibiting a lubricating property and a moiety having an epoxy group as a water-soluble or water-swellable polymer. Furthermore, in the case of using such a block copolymer, the epoxy groups of the block copolymer are cross-linked by a heating operation, and thereby a surface lubricating layer that is relatively difficult to peel off can be formed. However, a favorable lubricating property and excellent durability are in a trade-off relationship, and there is a demand for a technology that achieves both a favorable lubricating property and excellent durability.

Particularly, size reduction and diameter reduction of medical instruments in recent years have been in notable progress, and medical procedures allowing medical instruments with higher flexibility to approach narrow lesion sites in a living body have been spreading. Furthermore, along with complication of medical procedures, there are occasions where operation of medical instruments may be continued for a long period of time. Therefore, in order to maintain favorable maneuverability of a device even at a complicated lesion site, there is required a technology that further enhances a lubrication retaining property (durability) of the device surface compared to related art technologies. More specifically, a device having excellent sliding durability, which can maintain a high lubricating property even when sliding of the device surface is repeated, is required.

Therefore, there is a demand for a technology that can enhance durability (particularly, sliding durability) of a medical instrument and support medical procedures that are becoming more complicated and sophisticated.

The invention was achieved in view of the above-described circumstances, and it is an object of the invention to provide a method for manufacturing a medical instrument having a lubricating layer (coating layer) that exhibits excellent durability (particularly, sliding durability).

### Solution to Problem

The present inventors conducted a thorough investigation in order to solve the problems described above, and as a result, the inventors found that the object can be achieved by applying, on a base layer, a solution further including a choline derivative together with a block copolymer of a reactive monomer having an epoxy group and a hydrophilic monomer, thus completing the invention.

The present invention is defined by the independent claims, while the dependent claims concern the preferred embodiments. Any "embodiment" or "example" which is disclosed in the description but is not covered by the claims should be considered as presented for illustrative purpose only.

That is, the object is achieved by a method for manufacturing a medical instrument including a base layer and a lubricating layer carried on at least a portion of the base layer, the method comprising applying on the base layer a solution including: a block copolymer having a structural unit (A) derived from a reactive monomer having an epoxy group and a structural unit (B) derived from a hydrophilic monomer, a choline derivative, and a solvent, wherein the molar ratio between the structural unit (A) and the structural unit (B) of the block copolymer ranges from 1:10 to 1:30 and the choline derivative is at least one selected from the group consisting of a compound represented by Formula 1, as defined in independent claim 1, and a polymer having a structural unit represented by Formula 2, as defined in independent claim 1.

### Brief Description of Drawings

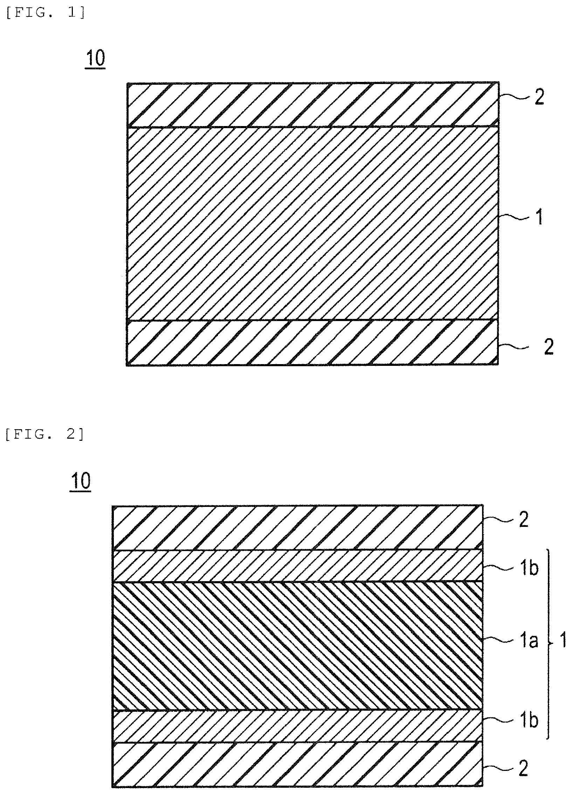

[Fig. 1] Fig. 1 is a partial cross-sectional view schematically showing a lamination configuration of a surface of a representative exemplary embodiment of a medical instrument manufactured by a method related to the invention. In Fig. 1, reference numeral 1 denotes a base layer; 2 denotes a lubricating layer; and 10 denotes a medical instrument.
[Fig. 2] Fig. 2 is a partial cross-sectional view schematically showing a configuration example in which the lamination configuration of the surface is different, as an application example of the exemplary embodiment of Fig. 1. In Fig. 2, reference numeral 1 denotes a base layer; 1a denotes a base layer core part; 1b denotes a base surface layer; 2 denotes a lubricating layer; and 10 denotes a medical instrument.

### Description of Embodiments

A method for manufacturing a medical instrument according to an embodiment of the invention is a method for manufacturing a medical instrument including a base layer and a lubricating layer carried on at least a portion of the base layer, the method comprising applying on the base layer a solution including: a block copolymer having a structural unit (A) derived from a reactive monomer having an epoxy group and a structural unit (B) derived from a hydrophilic monomer; a choline derivative; and a solvent, wherein the molar ratio between the structural unit (A) and the structural unit (B) of the block copolymer ranges from 1:10 to 1:30 and the choline derivative is at least one selected from the group consisting of a compound represented by Formula 1, as defined in independent claim 1, and a polymer having a structural unit represented by Formula 2, as defined in independent claim 1.

Furthermore, the medical instrument according to another embodiment of the invention is a medical instrument comprising a base layer and a lubricating layer carried on at least a portion of the base layer, the lubricating layer including a block copolymer having a structural unit (A) derived from a reactive monomer having an epoxy group and a structural unit (B) derived from a hydrophilic monomer; and a choline derivative, wherein the molar ratio between the structural unit (A) and the structural unit (B) of the block copolymer ranges from 1:10 to 1:30 and the choline derivative is at least one selected from the group consisting of a compound represented by Formula 1, as defined in independent claim 7, and a polymer having a structural unit represented by Formula 2, as defined in independent claim 7.

Incidentally, in the present specification, the structural unit (A) derived from a reactive monomer having an epoxy group is also simply referred to as a "structural unit derived from a reactive monomer" or a "structural unit (A)". Similarly, the structural unit (B) derived from a hydrophilic monomer is also simply referred to as a "structural unit derived from a hydrophilic monomer" or a "structural unit (B)". Furthermore, the block copolymer having the structural unit derived from a reactive monomer and the structural unit derived from a hydrophilic monomer is also simply referred to as a "block copolymer".

In the method for manufacturing a medical instrument according to the invention, a solution including a block copolymer having a structural unit derived from a reactive monomer and a structural unit derived from a hydrophilic monomer; a choline derivative; and a solvent is applied on a base layer. Furthermore, the medical instrument according to the invention comprises a base layer and a lubricating layer carried on at least a portion of the base layer, the lubricating layer including a block copolymer having a structural unit (A) derived from a reactive monomer having an epoxy group and a structural unit (B) derived from a hydrophilic monomer; and a choline derivative. Due to the configuration, a medical instrument manufactured by the method of the invention and the medical instrument according to the invention can exhibit excellent durability (surface lubrication retaining property and sliding durability).

The mechanism by which a medical instrument obtainable by the manufacturing method according to the invention and the medical instrument according to the invention can exhibit excellent durability is speculated as follows. Meanwhile, the invention is not limited to the following presumption.

According to the invention, first, a solution including a block copolymer having a structural unit (A) derived from a reactive monomer having an epoxy group and a structural unit (B) derived from a hydrophilic monomer; a choline derivative; and a solvent is applied on a base layer. As a result, a lubricating layer is formed on the base layer. The block copolymer that forms the lubricating layer exhibits a swelling property at the time of contact with a body fluid or an aqueous solvent and therefore imparts a lubricating property (surface lubricating property) to a medical instrument to reduce friction with luminal walls such as blood vessel walls.

On the other hand, it is conceived that the block copolymer forming the lubricating layer forms, after being applied as a solution, a reticulated structure (network) by cross-linking or polymerization with the epoxy group included in the structural unit (A) as a cross-linking point. Furthermore, it is conceived that the block copolymer is plasticized by addition of a choline derivative, is polymerized as blocks having similar properties aggregate with each other to form a network-like structure, is cross-linked by a subsequent heating treatment, and thus forms a reticulated structure (network). At this time, in this invention, it is conceived that the choline derivative included in a block copolymer solution promotes the cross-linking or polymerization (including formation of a network by a plurality of cross-links or formation of a network by aggregation). As a result, it is speculated that since the reticulated structure (network) in the lubricating layer can be formed at a higher density, a firm coating layer (lubricating layer) is formed, the firm coating layer can be maintained favorably even after sliding, and a high lubricating property (surface lubricating property) can be maintained for a longer period of time (that is, an excellent surface lubricating property can be maintained, and sliding durability can be enhanced). Incidentally, in the description below, cross-linking with the epoxy group as a cross-linking point included in the block copolymer such as described above or polymerization of the block copolymer may be simply referred to as "crosslinking or polymerization of the block copolymer".

Furthermore, depending on the type of the base layer (base), a functional group present at the surface of the base layer and the epoxy group included in the structural unit (A) may react and be bonded to each other. In this case, such a reaction is also promoted by the choline derivative, the epoxy group is bonded (fixed) also to the base layer, and thereby detachment from the base layer can be suppressed and prevented.

Therefore, a medical instrument manufactured by the method according to the invention and the medical instrument according to the invention have excellent durability (surface lubrication retaining property and sliding durability).

Hereinafter, embodiments of the invention will be described. Incidentally, the invention is not intended to be limited only to the following embodiments. Furthermore, the dimensional ratios of the drawings are exaggerated for the convenience of explanation and may be different from the actual ratios.

According to the present specification, the expression "X to Y" indicating a range includes X and Y and means "X or more and Y or less". Furthermore, unless particularly stated otherwise, operations and measurement of physical properties and the like are performed under the conditions of room temperature (20°C to 25°C) and a relative humidity of 40%RH to 50%RH.

### <Method for manufacturing medical instrument>

The method for manufacturing a medical instrument according to the invention includes applying, on a base layer, a solution including a block copolymer having a structural unit (A) derived from a reactive monomer having an epoxy group and a structural unit (B) derived from a hydrophilic monomer; a choline derivative; and a solvent ((I) solution application step, coating layer forming step). Furthermore, if necessary, drying and/or a heating treatment of the coating layer may also be carried out ((II) drying and/or heating treatment step), after the step (I). Moreover, washing of the lubricating layer that has been formed on the base ((III) washing step) may be carried out after the step (II). As such, a medical instrument having a lubricating layer (coating layer) that exhibits excellent durability is obtained by applying a solution including a choline derivative together with a block copolymer.

### (I) Solution application step (coating layer forming step)

In the method for manufacturing a medical instrument according to the invention, first, a solution including the above-described block copolymer, a choline derivative, and a solvent (in the present specification, also simply referred to as "block copolymer solution" or "coating liquid") is applied on a base layer ((I) solution application step, coating layer forming step). The solution application step is carried out for the purpose of carrying (or coating) a lubricating layer including a block copolymer on at least a portion of the surface of a base layer. Incidentally, the term "carrying" means a state in which the lubricating layer is fixed such that the lubricating layer is not easily separated from the surface of the base layer, and is intended to include not only a form in which the entire surface of the base layer is completely covered by the lubricating layer but also a form in which only a portion of the base surface is covered by the lubricating layer, that is, a form in which the lubricating layer is attached only to a portion of the base surface. Accordingly, the method of applying the solution is not particularly limited, except that the solution including the block copolymer having a structural unit (A) and a structural unit (B), a choline derivative, and a solvent is used, and the method can be applied in the same manner as in a known method, or by appropriately modifying this known method.

In the solution application step, specifically, a solution (coating liquid) is prepared by dissolving the block copolymer and the choline derivative in a solvent, and a coating layer is formed by coating a base with the solution (coating liquid).

Hereinafter, preferred embodiments of the (I) solution application step (coating layer forming step) will be described in detail.

### <<Preparation of block copolymer solution (coating liquid) >>

As described above, in the solution application step (coating layer forming step), in order to apply a solution including a block copolymer, a choline derivative, and a solvent on a base layer, first, a block copolymer solution (coating liquid) is prepared.

### (Block copolymer)

According to the invention, the block copolymer forms the lubricating layer that is carried on at least a portion of the base layer. That is, with regard to the medical instrument obtainable by the method according to the invention, the lubricating layer includes the block copolymer.

The block copolymer according to the invention has a structural unit (A) derived from a reactive monomer having an epoxy group and a structural unit (B) derived from a hydrophilic monomer.

The reactive monomer having an epoxy group, which constitutes the block copolymer, has an epoxy group as a reactive group. By introducing such a structural unit (A) derived from a reactive monomer into the block copolymer, the block copolymer is cross-linked or polymerized with the same block copolymer by means of the epoxy group, and thus a reticulated structure is formed. Here, as described above, as a choline derivative is added, cross-linking or polymerization of the block copolymer is promoted, and thus, strength of the lubricating layer can be enhanced. Specifically, it is speculated that the choline derivative contributes to polymerization of the block copolymer not only by promoting cross-linking of epoxy groups but also by plasticizing the block copolymer, thereby promoting aggregation of blocks having similar properties. Therefore, the medical instrument obtainable by the method according to the invention has excellent sliding durability and can maintain the shape favorably even after sliding. Furthermore, depending on the type of the base layer, the lubricating layer can be firmly bonded (fixed) to the base layer via an epoxy group. Here, as the choline derivative is added, bonding between the lubricating layer and the base layer by means of epoxy groups is also promoted, and therefore, detachment from the base layer can be further suppressed and prevented. Therefore, even from such a point of view, the medical instrument obtainable by the method according to the invention exhibits further enhanced sliding durability.

Incidentally, formation of a reticulated structure (network) by the structural unit (A) that constitutes the block copolymer can be checked by a known method; however, in the present specification, the formation of the reticulated structure (network) can be checked by disappearance of an epoxy group and formation of an ether bond by ATR-IR. Meanwhile, regarding specific measurement conditions for ATR-IR, the conditions described in Examples can be employed.

The reactive monomer that constitutes the block copolymer is not particularly limited as long as it has an epoxy group, and any known compound can be used. Above all, from the viewpoint that crosslinking or polymerization of the block copolymer is easily controllable, it is preferable that the reactive monomer having an epoxy group includes at least one selected from the group consisting of glycidyl acrylate, glycidyl methacrylate (GMA), 3,4-epoxycyclohexylmethyl acrylate, 3,4-epoxycyclohexylmethyl methacrylate, β-methylglycidyl methacrylate, and allyl glycidyl ether. Above all, when the enhancement of the property of easily forming a reticulated structure, ease of manufacture, and the like are considered, glycidyl (meth)acrylate is preferred, and glycidyl methacrylate (GMA) is more preferred. Incidentally, in the present specification, the term "(meth)acrylate" includes both acrylate and methacrylate.

Regarding the reactive monomer, one kind may be used alone, or two or more kinds may be used in combination. That is, a reactive site derived from the reactive monomer may be a homopolymer type composed of one kind of reactive monomer alone, or may be a copolymer type composed of two or more kinds of the reactive monomers described above. Incidentally, the form of the reactive site in the case of using two or more kinds may be a block copolymer or may be a random copolymer.

The hydrophilic monomer that constitutes the block copolymer has a swelling property at the time of contact with a body fluid or an aqueous solvent and therefore imparts a lubricating property (surface lubricating property) to a medical instrument. Accordingly, by introducing such a structural unit (B) derived from a hydrophilic monomer into the block copolymer, the lubricating property (surface lubricating property) of the medical instrument is enhanced, and the friction occurring when the medical instrument comes into contact with luminal walls such as blood vessel walls can be reduced.

The hydrophilic monomer that constitutes the block copolymer is not particularly limited as long as it has the above-described characteristics, and any known compound can be used. Examples thereof include acrylamide and derivatives thereof, vinylpyrrolidone, acrylic acid or methacrylic acid and derivatives thereof, polyethylene glycol acrylate and derivatives thereof, a monomer having a sugar or a phospholipid in a side chain, and a water-soluble monomer such as maleic anhydride. More specific examples include acrylic acid, methacrylic acid, N-methylacrylamide, N,N-dimethylacrylamide (DMAA), acrylamide, acryloylmorpholine, N,N-dimethylaminoethyl acrylate, N-vinylpyrrolidone, 2-methacryloyloxyethyl phosphorylcholine, 2-methacryloyloxyethyl-D-glycoside, 2-methacryloyloxyethyl-D-mannoside, vinyl methyl ether, 2-hydroxyethyl (meth)acrylate, 4-hydroxybutyl (meth)acrylate, 2-hydroxypropyl (meth)acrylate, 2-hydroxybutyl (meth)acrylate, 6-hydroxyhexyl (meth)acrylate, 1,4-cyclohexanedimethanol mono(meth)acrylate, 1-chloro-2-hydroxypropyl (meth)acrylate, diethylene glycol mono(meth)acrylate, 1,6-hexanediol mono(meth)acrylate, pentaerythritol tri(meth)acrylate, dipentaerythritol penta (meth) acrylate, neopentyl glycol mono(meth)acrylate, trimethylolpropane di(meth)acrylate, trimethylolethane di(meth)acrylate, 2-hydroxy-3-phenyloxypropyl (meth)acrylate, 4-hydroxycyclohexyl (meth)acrylate, 2-hydroxy-3-phenyloxy (meth)acrylate, 4-hydroxycyclohexyl (meth)acrylate, cyclohexanedimethanol mono(meth)acrylate, poly(ethylene glycol) methyl ether acrylate, and poly(ethylene glycol) methyl ether methacrylate. From the viewpoints of impartation of an excellent lubricating property, ease of synthesis, and maneuverability, it is preferable that the hydrophilic monomer includes at least one selected from the group consisting of N,N-dimethylacrylamide, acrylamide, 2-hydroxyethyl methacrylate, and N-vinylpyrrolidone. Above all, from the viewpoint of having an excellent lubricating property, the hydrophilic monomer is preferably N,N-dimethylacrylamide (DMAA).

Regarding the hydrophilic monomer, one kind may be used alone, or two or more kinds may be used in combination. That is, the hydrophilic site derived from a hydrophilic monomer may be a homopolymer type composed of one kind of hydrophilic monomer alone, or may be a copolymer type composed of two or more kinds of the hydrophilic monomers. Meanwhile, the form of the hydrophilic site in the case of using two or more kinds may be a block copolymer or may be a random copolymer.

The block copolymer has the structural unit (A) and the structural unit (B). According to the claimed invention, the ratio between the structural unit (A) and the structural unit (B) (molar ratio of structural unit (A) : structural unit (B)) is 1 : 10 to 1 : 30. When the ratio is in such a range, the lubricating layer can sufficiently exhibit a lubricating property due to the structural unit (B) and can exhibit sufficient strength of the coating layer (lubricating layer), a bonding property to the base layer, and durability due to the structural unit (A). Meanwhile, the molar ratio of the structural unit (A) : structural unit (B) can be controlled by adjusting the feed ratio (molar ratio) of the various monomers in the stage of manufacturing the block copolymer. At this time, the feed ratio (molar ratio) between the reactive monomer having an epoxy group and the hydrophilic monomer in the stage of manufacturing the block copolymer is 1 : 10 to 1 : 30. Incidentally, the molar ratio of structural unit (A) : structural unit (B) can be checked by, for example, performing NMR measurement (¹H-NMR measurement, ¹³C-NMR measurement, and the like) and ATR-IR measurement for the copolymer.

The weight average molecular weight (Mw) of the block copolymer is preferably 10,000 to 10,000,000, in view of solubility. From the viewpoint of the ease of preparing a block copolymer solution (coating liquid), the weight average molecular weight of the block copolymer is more preferably 100,000 to 10,000,000. Regarding the "weight average molecular weight" in the present specification, a value measured by Gel Permeation Chromatography (GPC) by using polystyrenes as standard materials is employed.

Furthermore, the method for manufacturing the block copolymer is not particularly limited, and the block copolymer can be produced by applying a conventionally known polymerization method such as, for example, a living radical polymerization method, a polymerization method using a macroinitiator, or a polycondensation method. Among these, from the viewpoint that the molecular weight and the molecular weight distribution of the structural unit (site) derived from a reactive monomer and the structural unit (site) derived from a hydrophilic monomer are easily controllable, a living radical polymerization method or a polymerization method using a macroinitiator is preferably used. The living radical polymerization method is not particularly limited; however, for example, the methods described in JP-A-11-263819, JP-A-2002-145971, JP-A-2006-316169, and the like, and an atom transfer radical polymerization (ATRP) method and the like can be applied in the same manner or after being appropriately modified. Furthermore, in the polymerization method using a macroinitiator, for example, a macroinitiator having a reactive site having a reactive functional group, and a radical polymerizable group such as a peroxide group is produced, subsequently the macroinitiator and a monomer for forming a hydrophilic site are polymerized in a polymerization solvent, and thereby a block copolymer having a hydrophilic site and a reactive site can be produced.

The polymerization solvent is not particularly limited; however, for example, aliphatic organic solvents such as n-hexane, n-heptane, n-octane, n-decane, cyclohexane, methylcyclohexane, and liquid paraffin; ether-based solvents such as tetrahydrofuran and dioxane; aromatic organic solvents such as benzene, toluene, and xylene; halogen-based organic solvents such as 1,2-dichloroethane and chlorobenzene; and aprotic polar organic solvents such as N,N-dimethylformamide and dimethyl sulfoxide, can be used. Incidentally, the solvents can be used singly or as mixtures of two or more kinds thereof.

With regard to the polymerization, the polymerization conditions are also not particularly limited as long as the copolymerization proceeds. For example, it is preferable to set the polymerization temperature preferably to 30°C to 150°C, and more preferably 40°C to 100°C. The polymerization time is preferably 30 minutes to 30 hours, and more preferably 3 to 24 hours.

Furthermore, at the time of manufacturing the block copolymer, a chain transfer agent, a polymerization rate adjusting agent, a surfactant, a water-soluble polymer, a water-soluble inorganic compound (an alkali metal salt, an alkali metal hydroxide, a polyvalent metal salt, a non-reducing alkali metal salt pH buffering agent, and the like), an inorganic acid, an inorganic acid salt, an organic acid, an organic acid salt, and other additives may also be appropriately used as necessary.

It is preferable that the block copolymer after copolymerization is purified by a general purification method such as a reprecipitation method, a dialysis method, an ultrafiltration method, or an extraction method.

### (Choline derivative)

According to the invention, the choline derivative promotes cross-linking or polymerization of the block copolymer. As a result, a firm lubricating layer can be formed, and excellent durability (sliding durability) can be obtained.

Promotion of cross-linking or polymerization of the block copolymer such as described above can also be induced by addition of a strong acid. However, the present inventors found that when a strong acid is added, the cross-linking or polymerization proceeds rapidly in the block copolymer solution (coating liquid), and as a result, the viscosity of the coating liquid increases so that it is difficult to uniformly apply the coating liquid. In contrast, since the choline derivative mildly promotes cross-linking or polymerization of the block copolymer, an increase in the viscosity of the coating liquid caused by cross-linking or polymerization of the block copolymer in the coating liquid such as described above can be suppressed. Accordingly, in this invention, the coating liquid can be uniformly applied by using a choline derivative. Furthermore, in the case of using a strong acid, it is necessary to separately prepare a solution of the block copolymer and a solution of the strong acid and apply these solutions in order due to the reasons described above; however, according to the invention, since it is sufficient to apply a liquid including both the block copolymer and the choline derivative when forming the lubricating layer (coating layer), there is an advantage that formation of the lubricating layer (coating layer) by one liquid is enabled.

According to the present invention, with the purpose of further enhancing sliding durability, the choline derivative is at least one selected from the group consisting of a compound represented by the following Formula 1 and a polymer having a structural unit represented by the following Formula 2.

### <<Compound represented by Formula 1>>

The compound represented by the following Formula 1 may be of one kind alone or may be of two or more kinds.
[Chem 1]

Formula 1 **R₁-O-(CH₂)₂-N⁺(CH₃)₃X₁⁻**

In Formula 1, X₁⁻ represents a halide ion, an organic acid ion, or an inorganic acid ion.

Examples of the halide ion include fluoride ion, chloride ion, bromide ion, and iodide ion.

Examples of the organic acid ion include ions of carboxylic acids such as acetic acid, propionic acid, butyric acid, valeric acid, caproic acid, enanthic acid, caprylic acid, pelargonic acid, capric acid, lauric acid, myristic acid, palmitic acid, margaric acid, stearic acid, oleic acid, linoleic acid, linolenic acid, arachidonic acid, docosahexaenoic acid, eicosapentaenoic acid, oxalic acid, malonic acid, succinic acid, benzoic acid, phthalic acid, isophthalic acid, terephthalic acid, salicylic acid, mandelic acid, gallic acid, mellitic acid, cinnamic acid, pyruvic acid, lactic acid, malic acid, citric acid, fumaric acid, tartaric acid, maleic acid, aconitic acid, glutaric acid, and adipic acid; and ions of alkylsulfuric acids such as dimethylsulfuric acid and diethylsulfuric acid. Incidentally, in a case where the ion of a carboxylic acid is an ion of a polyvalent carboxylic acid, the ion may be in a form in which protons of some carboxyl groups are not dissociated, as in the case of dihydrogen citrate ion, hydrogen tartrate ion (bitartrate ion), or the like.

Examples of the inorganic acid ion include hydrogen sulfate ion (HSO₄⁻); sulfate ion (SO₄²⁻); nitrate ion (NO₃⁻); dihydrogen phosphate ion (H₂PO₄⁻), hydrogen phosphate ion (HPO₄²⁻), phosphate ion (PO₄³⁻): and perchlorate ion (ClO₄⁻).

In Formula 1, X₁⁻ is preferably a halide ion or an organic acid ion from the viewpoint of sliding durability, and from the viewpoint of making the removal by washing easier and further enhancing the sliding durability, X₁⁻ is more preferably a halide ion, even more preferably chloride ion, bromide ion, or iodide ion, and particularly preferably chloride ion.

In Formula 1, R₁ represents a hydrogen atom, a substituted or unsubstituted alkyl group having 1 to 12 carbon atoms, or Rₐ-C(=O)-, where Rₐ represents a substituted or unsubstituted alkyl group having 1 to 12 carbon atoms, a substituted or unsubstituted aryl group having 6 to 12 carbon atoms, CH₂=CH-, or CH₂=C(CH₃)-.

The alkyl group having 1 to 12 carbon atoms may be either in a straight chain form or a branched chain form, and examples include a methyl group, an ethyl group, an n-propyl group, an isopropyl group, an n-butyl group, an isobutyl group, a sec-butyl group, a tert-butyl group, an n-pentyl group, an isopentyl group, a neopentyl group, an n-hexyl group, and a cyclohexyl group. Among these, a straight-chain or branched-chain alkyl group having 1 to 8 carbon atoms is preferred, a straight-chain alkyl group having 1 to 3 carbon atoms is more preferred, and a methyl group is particularly preferred.

Examples of the aryl group having 6 to 12 carbon atoms include a phenyl group and a naphthyl group. Among these, a phenyl group is preferred.

A substituent that may be present in the alkyl group having 1 to 12 carbon atoms and an aryl group having 6 to 12 carbon atoms is not particularly limited as along as the effects of the invention are provided. Examples of the substituent include, for example, an alkyl group, an alkenyl group, an alkynyl group, an aryl group, an acyl group, an alkoxycarbonyl group, an alkoxy group, an aryloxy group, an aryloxycarbonyl group, and an acyloxy group. However, an alkyl group having 1 to 12 carbon atoms shall not be substituted with an alkyl group.

From the viewpoint of making the removal by washing easier, Rₐ is preferably a methyl group, an ethyl group, an n-propyl group, a phenyl group, CH₂=CH-, or CH₂=C(CH₃)-, and more preferably a methyl group, a phenyl group, CH₂=CH-, or CH₂=C(CH₃)-. That is, Rₐ-C(=O)- is preferably an acetyl group, a propionyl group, a butyryl group, a benzoyl group, an acryloyl group, or a methacryloyl group, and more preferably an acetyl group, a benzoyl group, an acryloyl group, or a methacryloyl group.

In Formula 1, R₁ is preferably a hydrogen atom or Rₐ-C(=O)- from the viewpoint of the manufacturing cost, and R₁ is more preferably a hydrogen atom from the viewpoint of further enhancing sliding durability.

Examples of the compound represented by the Formula 1 include choline chloride, choline bromide, choline iodide, choline dihydrogen citrate, choline bitartrate, acetylcholine chloride, benzoylcholine chloride, acryloylcholine chloride, and methacryloylcholine chloride. Above all, from the viewpoint of further enhancing sliding durability, at least one selected from the group consisting of choline chloride, choline bromide, choline iodide, acetylcholine chloride, benzoylcholine chloride, and acryloylcholine chloride is preferred, and choline chloride is particularly preferred.

The compound represented by Formula 1 may be a synthetic product or a commercially available product. Commercially available products can be purchased from Tokyo Chemical Industry Co., Ltd., Sigma-Aldrich Corporation, Fujifilm Wako Pure Chemical Corp., and the like.

### <<Polymer having structural unit represented by Formula 2>>

The polymer having a structural unit represented by the following Formula 2 may be of one kind alone or may be of two or more kinds. In the case of the latter, each structural unit may exist in the form of a block or may exist in a random form.

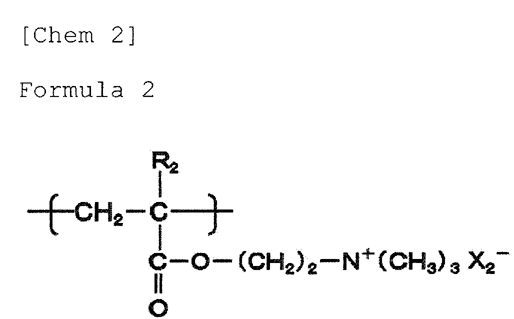

In Formula 2, X₂⁻ represents a halide ion, an organic acid ion, or an inorganic acid ion. Examples of the halide ion, the organic acid ion, and the inorganic acid ion are as described above. Above all, X₂⁻ is preferably a halide ion or an organic acid ion from the viewpoint of sliding durability, and from the viewpoint of making the removal by washing easier and further enhancing sliding durability, X₂⁻is more preferably a halide ion, even more preferably chloride ion, bromide ion, or iodide ion, and particularly preferably chloride ion.

In Formula 2, R₂ represents a hydrogen atom or a methyl group, and from the viewpoint of ease of manufacture, R₂ is preferably a hydrogen atom.

Therefore, according to an exemplary embodiment of the invention, the structural unit represented by the Formula 2 is preferably a structural unit derived from at least one of acryloylcholine chloride and methacryloylcholine chloride, and more preferably a structural unit derived from acryloylcholine chloride.

That is, in a preferred embodiment of the invention, in the method for manufacturing the medical instrument of the invention, the choline derivative is at least one selected from the group consisting of a compound represented by the following Formula 1 and a polymer having a structural unit represented by the following Formula 2:
[Chem 3]

Formula 1 **R₁-O-(CH₂)₂-N⁺(CH₃)₃ X₁⁻**

In Formula 1,
X₁⁻ represents a halide ion or an organic acid ion, and
R₁ represents a hydrogen atom or Rₐ-C(=O)-, where Rₐ represents a substituted or unsubstituted alkyl group having 1 to 12 carbon atoms, a substituted or unsubstituted aryl group having 6 to 12 carbon atoms, CH₂=CH-, or CH₂=C(CH₃)-;

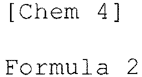

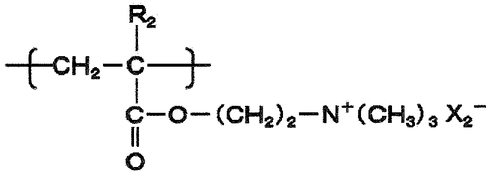

In Formula 2,
X₂⁻ represents a halide ion or an organic acid ion, and
R₂ represents a hydrogen atom or a methyl group.

The polymer having a structural unit represented by the Formula 2 may have only a structural unit represented by the Formula 2 and may further have a structural unit other than the structural unit represented by the Formula 2 (hereinafter, also referred to as "other structural unit"), in addition to the structural unit represented by the Formula 2. In the case of the latter, the molar ratio of the structural unit represented by the Formula 2 and the other structural unit may be, for example, 1 : 1 to 1 : 30, and may be 1 : 10 to 1 : 20. Incidentally, this molar ratio can be checked by performing, for example, NMR measurement (¹H-NMR measurement, ¹³C-NMR measurement, or the like), ATR-IR measurement, and the like for the copolymer. In a case where the polymer having a structural unit represented by the Formula 2 has two or more kinds of other structural units, the composition (molar ratio) of the other structural units intends to mean the total content of the other structural units.

The other structural unit is not particularly limited; however, from the viewpoint of imparting an excellent lubricating property, the other structural unit is preferably a structural unit derived from the hydrophilic monomer described in the section (Block copolymer). Examples of the hydrophilic monomer include those listed as examples in the section (Block copolymer), and at least one selected from the group consisting of N,N-dimethylacrylamide, acrylamide, 2-hydroxyethyl methacrylate, and N-vinylpyrrolidone, while N,N-dimethylacrylamide (DMAA) is even more preferred. The hydrophilic monomer may be of one kind alone or may be of two or more kinds.

Therefore, regarding the polymer having a structural unit represented by Formula 2, for example, a homopolymer of acryloylcholine chloride (poly(acryloylcholine chloride)), a homopolymer of methacryloylcholine chloride (poly(methacryloylcholine chloride)), a copolymer of acryloylcholine chloride and N,N-dimethylacrylamide, a copolymer of methacryloylcholine chloride and N,N-dimethylacrylamide, and a copolymer of acryloylcholine chloride, methacryloylcholine chloride, and N,N-dimethylacrylamide are preferred. These may be used singly or in combination of two or more kinds.

In a case where the polymer having a structural unit represented by Formula 2 is a copolymer, the polymer may be a block copolymer or a random copolymer.

A weight average molecular weight (Mw) of the polymer having a structural unit represented by Formula 2 is preferably 50,000 to 800,000, more preferably 100,000 to 500,000, and particularly preferably 150,000 to 350,000, from the viewpoint of sliding durability.

The polymer having a structural unit represented by Formula 2 may be a synthetic product or a commercially available product.

The method for manufacturing the polymer having a structural unit represented by Formula 2 is not particularly limited, and the polymer can be produced by applying a conventionally known polymerization method such as a bulk polymerization method, a suspension polymerization method, an emulsion polymerization method, a solution polymerization method, a living radical polymerization method, a polymerization method using a macroinitiator, or a polycondensation method.

In the above-described method, the polymerization solvent that can be used for the preparation of a monomer solution is not particularly limited as long as the polymerization solvent can dissolve the monomers used as described above. Examples include aqueous solvents such as water, alcohols such as methanol, ethanol, propanol, and isopropanol, and polyethylene glycols; aromatic solvents such as toluene, xylene, and tetralin; and halogen-based solvents such as chloroform, dichloroethane, chlorobenzene, dichlorobenzene, and trichlorobenzene. Furthermore, a monomer concentration in the monomer solution is not particularly limited; however, the total concentration of a monomer in the monomer solution is usually 15% to 60% by mass.

The polymerization initiator is not particularly limited, and any known polymerization initiator may be used. The polymerization initiator is preferably a radical polymerization initiator from the viewpoint of having excellent polymerization stability, and specific examples include persulfates such as potassium persulfate (KPS), sodium persulfate, and ammonium persulfate; peroxides such as hydrogen peroxide, t-butyl peroxide, and methyl ethyl ketone peroxide; and azo compounds such as azobisisobutyronitrile (AIBN), 2,2'-azobis(4-methoxy-2,4-dimethylvaleronitrile), 2,2'-azobis(2,4-dimethylvaleronitrile), 2,2'-azobis[2-(2-imidazolin-2-yl)propane] dihydrochloride, 2,2'-azobis[2-(2-imidazolin-2-yl)propane] disulfate dihydrate, 2,2'-azobis(2-methylpropionamidine) dihydrochloride, 2,2'-azobis[N-(2-carboxyethyl)-2-methylpropionamidine)] hydrate, 3-hydroxy-1,1-dimethylbutyl peroxyneodecanoate, α-cumyl peroxyneodecanoate, 1,1,3,3,-tetrabutyl peroxyneodecanoate, t-butyl peroxyneodecanoate, t-butyl peroxyneoheptanoate, t-butyl peroxypivalate, t-amyl peroxyneodecanoate, t-amyl peroxypivalate, di(2-ethylhexyl) peroxydicarbonate, di(secondary-butyl) peroxydicarbonate, and azobiscyanovaleric acid. Furthermore, for example, the radical polymerization initiator may be combined with a reducing agent such as sodium sulfite, sodium hydrogen sulfite, or ascorbic acid and may be used as a Redox initiator. An amount of the polymerization initiator incorporated is preferably 0.5 to 5 mmol with respect to 1 mol of the total amount of monomers. When such an amount of the polymerization initiator incorporated is adopted, polymerization of monomers proceeds efficiently.

The polymerization initiator may be directly mixed with monomers and a polymerization solvent, or may be mixed in the form of a solution having the monomers dissolved in advance in another solvent, with monomers and a polymerization solvent.

In the case of using the polymerization initiator in the form of a solution, a solution obtained by dissolving monomers in a polymerization solvent may be subjected to degassing in advance before the addition of the polymerization initiator solution. Regarding the degassing treatment, for example, the solution may be subjected to bubbling with an inert gas such as nitrogen gas or argon gas for about 10 seconds to 5 hours. During the degassing treatment, a temperature of the solution may be regulated to about 30°C to 80°C, and preferably the polymerization temperature in the following polymerization step.

Next, the monomers are polymerized by heating the monomer solution. Here, regarding the polymerization method, for example, any known polymerization methods such as radical polymerization, anionic polymerization, and cationic polymerization can be employed, and preferably, radical polymerization which facilitates manufacture is used.

The polymerization conditions are not particularly limited as long as the conditions are conditions in which the monomers can be polymerized. Specifically, a polymerization temperature is preferably 30°C to 80°C, and more preferably 40°C to 55°C. Furthermore, a polymerization time is preferably 1 to 24 hours, and preferably 5 to 12 hours.

Furthermore, if necessary, a chain transfer agent, a polymerization rate adjusting agent, a surfactant, and other additives may be appropriately used during polymerization.

The atmosphere in which the polymerization reaction is carried out is not particularly limited, and the polymerization reaction can be carried out in an air atmosphere, an inert gas atmosphere such as nitrogen gas or argon gas, or the like. Furthermore, during the polymerization reaction, the reaction liquid may be stirred.

The polymer after polymerization can be purified by general purification methods such as a reprecipitation method (precipitation method), a dialysis method, an ultrafiltration method, and an extraction method.

The polymer after purification can be dried by any method such as freeze-drying, drying under reduced pressure, spray drying, or heat drying; however, from the viewpoint that the influence on the physical properties of the polymer is small, freeze-drying or drying under reduced pressure is preferred.

### (Solvent)

In the invention, for the purpose of uniformly carrying a block copolymer on a base layer, a uniform solution including the above-described block copolymer and the above-described choline derivative is prepared, and this solution (coating liquid) is applied on the base layer.

A solvent used for dissolving the block copolymer and the choline derivative according to the invention is not particularly limited as long as it can dissolve the block copolymer and the choline derivative according to the invention. Specifically, examples include water; alcohols such as methanol, ethanol, isopropanol, and ethylene glycol; ketones such as acetone, methyl ethyl ketone, and cyclohexanone; esters such as ethyl acetate; halides such as chloroform; olefins such as hexane; ethers such as tetrahydrofuran (THF) and butyl ether; aromatics such as benzene and toluene; amides such as N,N-dimethylformamide (DMF); and sulfoxides such as dimethyl sulfoxide (DMSO); however, the examples are not limited to these. Regarding these, one kind may be used alone, or two or more kinds may be used in combination. Above all, from the viewpoint that the solvent can uniformly dissolve the block copolymer and the choline derivative and application of the solution can be uniformly performed, as the solvent for the solution (coating liquid), an amide such as DMF, and a sulfoxide such as DMSO are preferred, and DMF and DMSO are particularly preferred.

### (Other components)

The block copolymer solution (coating liquid) for forming the lubricating layer may include other components in addition to the block copolymer, the choline derivative, and the solvent. The other components are not particularly limited, and in a case where the medical instrument such as a catheter is intended to be inserted into a lumen in a living body or a tube cavity, examples include drugs (physiologically active substances) such as an anticancer agent, an immunosuppressant, an antibiotic substance, an antirheumatic drug, an antithrombotic drug, an HMG-CoA reductase inhibitor, an ACE inhibitor, a calcium antagonist, an antihyperlipidemic drug, an integrin inhibitory drug, an antiallergic agent, an antioxidant, a GPIIb/IIIa antagonist, a retinoid, a flavonoid, a carotenoid, a lipid improving drug, a DNA synthesis inhibitor, a tyrosine kinase inhibitor, an antiplatelet drug, a vascular smooth muscle growth inhibitory drug, an anti-inflammatory drug, a biological material, an interferon, and an NO production promoting substance. Here, an amount of the other components added is not particularly limited, and a commonly used amount is similarly applied. Ultimately, the amount of the other components added is appropriately selected in consideration of severity of disease to which the component is applied, a weight of a patient, and the like.

### (Preparation of block copolymer solution (coating liquid))

The block copolymer solution (coating liquid) is prepared using the block copolymer, the choline derivative, and the solvent. An order of addition and a method of addition of the above-described various components are not particularly limited. The various components may be added all at once or separately in a stepwise or continuous manner. Furthermore, a mixing method is also not particularly limited, and any known method can be used. A preferable method for preparing the block copolymer solution (coating liquid) includes sequentially adding a block copolymer and a choline derivative into a solvent and stirring the mixture in the solvent.

A concentration of the block copolymer in the block copolymer solution (coating liquid) is not particularly limited. From the viewpoint of further enhancing coatability, and a lubricating property and durability of the lubricating layer, the concentration of the block copolymer in this solution (coating liquid) is preferably 0.01% to 20% by mass, more preferably 0.05% to 15% by mass, and particularly preferably 1% to 10% by mass. When the concentration of the block copolymer is in the range described above, the lubricating property and durability of the lubricating layer to be obtained can be sufficiently exhibited. That is, according to a preferred embodiment of the invention, the solution (block copolymer solution, coating liquid) includes 0.01% to 20% by mass (more preferably 0.05% to 15% by mass, and particularly preferably 1% to 10% by mass) of the block copolymer. Furthermore, a uniform lubricating layer having a desired thickness can be easily obtained by one-time coating, and a viscosity of the solution falls in an appropriate range, which is preferable from the viewpoints of maneuverability (for example, ease of coating) and production efficiency. However, even when the concentration is out of the range, the solution can be sufficiently utilized as long as the concentration is in a range that does not affect the operating effect of the invention.

A concentration of the choline derivative in the block copolymer solution (coating liquid) is also not particularly limited. From the viewpoint of causing the cross-linking or polymerization of the block copolymer to sufficiently proceed but not to excessively proceed (cross-linking or polymerization can be appropriately promoted), the concentration of the choline derivative in the solution (coating liquid) is preferably 0.005% to 20% by mass, and more preferably 0.01% to 10% by mass. When the concentration of the choline derivative is in the above-described range, the lubricating property and durability of the lubricating layer to be obtained can be sufficiently exhibited. However, even when the concentration is out of the range, the block copolymer solution can be sufficiently utilized as long as the concentration does not affect the operating effect of the invention. Above all, from the viewpoint of further enhancing sliding durability, the concentration is particularly preferably 0.06% to 2% by mass.

In addition, a mixing ratio of the block copolymer and the choline derivative in the block copolymer solution (coating liquid) is not particularly limited; however, from the viewpoint of further enhancing coatability, and a lubricating property and durability of the lubricating layer, it is preferable that this solution (coating liquid) includes the block copolymer and the choline derivative at a mass ratio of 1 : 0.002 to 1 : 2, and more preferably at a mass ratio of 1 : 0.005 to 1 : 1. Above all, from the viewpoint of further enhancing sliding durability, it is even more preferable that the solution includes them at a mass ratio of 1 : 0.01 to 1 : 0.2. When the mass ratio of the block copolymer and the choline derivative is in the range described above, the lubricating property and durability of the lubricating layer to be obtained can be sufficiently exhibited.

### <<Application of block copolymer solution (coating liquid) >>

Next, after a solution (coating liquid) including the block copolymer, the choline derivative, and the solvent is prepared as described above, the solution is applied on a base layer.

The base layer may be constructed from any material; however, examples include metal materials, polymer materials (resin materials), and ceramics.

Among the materials constituting the base layer, the metal materials are not particularly limited, and metal materials that are generally used for medical instruments such as a catheter, a guide wire, and an indwelling needle are used. Specifically, various stainless steels such as SUS304, SUS314, SUS316, SUS316L, SUS420J2, and SUS630; gold, platinum, silver, copper, nickel, cobalt, titanium, iron, aluminum; and various alloys such as a tin- or nickel-titanium alloy, a nickel-cobalt alloy, a cobalt-chromium alloy, and a zinc-tungsten alloy may be mentioned. These may be used singly or in combination of two or more kinds thereof.

Regarding the metal materials described above, a metal material optimal for the base layer of a catheter, a guide wire, an indwelling needle, or the like, which are use applications, may be appropriately selected.

Furthermore, among the materials constituting the base layer, the polymer materials (resin materials or elastomer materials) are not particularly limited, and polymer materials that are generally used for medical instruments such as a catheter, an introducer, a guide wire, and an indwelling needle are used. Specifically, examples include a polyamide resin, polyolefin resins such as a polyethylene resin and a polypropylene resin, a modified polyolefin resin, a cyclic polyolefin resin, an epoxy resin, a polyurethane resin, a diallyl phthalate resin (allyl resin), a polycarbonate resin, a fluororesin, amino resins (a urea resin, a melamine resin, and a benzoguanamine resin), polyester resins such as a polyethylene terephthalate resin and a polybutylene terephthalate resin, a styrol resin, an acrylic resin, a polyacetal resin, a vinyl acetate resin, a phenol resin, a vinyl chloride resin, a silicone resin (silicon resin), a polyether resin, and a polyimide resin.

Furthermore, thermoplastic elastomers such as a polyurethane elastomer, a polyester elastomer, and a polyamide elastomer can also be used as the material for the base layer.

These polymer materials may be used singly, or may be used as a mixture of two or more kinds or a copolymer of two or more kinds of monomers constituting any of the resin or elastomer described above. Above all, as the polymer material, a polyethylene resin, a polyurethane resin, a polyethylene terephthalate resin, a polyamide resin, and a polyamide elastomer are preferred, and a polyethylene resin, a polyamide resin, and a polyamide elastomer are more preferred. A carboxy group or an amino group as a terminal group included in the polyamide resin or a polyamide elastomer can be subjected to a cross-linking reaction with an epoxy group in the block copolymer. Furthermore, it can be said that these polymer materials (particularly, a polyamide resin and a polyamide elastomer) are relatively soft, and the block copolymer constituting the lubricating layer can easily infiltrate into the polymer materials. Therefore, the bonding property of the polymer materials (particularly, a polyamide resin and a polyamide elastomer) and the block copolymer increase, and thus a lubricating layer having superior durability can be formed. Regarding the polymer materials, a polymer material optimal as the base layer of a catheter, a guide wire, an indwelling needle, or the like, which are use applications, may be appropriately selected.

In the method for manufacturing a medical instrument according to the invention, as will be described below, even when the block copolymer solution (coating liquid) is applied and then maintained at a relatively low temperature, cross-linking or polymerization of the block copolymer proceeds, and a lubricating layer having excellent durability can be formed. In other words, an effect that the temperature at which the lubricating layer is fixed onto the base layer can be lowered is also provided. Therefore, in the method for manufacturing a medical instrument according to the invention, a polymer material (resin material or an elastomer material) is suitably used as a constituent material for the base layer. According to the invention, since the lubricating layer can be fixed at a low temperature, even when a polymer material that is easily deformed or plasticized by heat is included in the base layer, deformation or plasticization of the base layer is suppressed, and dimensional stability is enhanced.

A shape of the base layer is not particularly limited and a shape such as a sheet shape, a linear shape (wire), or a tubular shape (tube) is appropriately selected according to the use mode.

A method of applying (coating) the block copolymer solution (coating liquid) on the surface of a base layer is not particularly limited, and conventionally known methods such as an application and printing method, an immersion method (a dipping method, a dip coating method), a spraying method (spray method), a spin coating method, a mixed solution-impregnated sponge coating method, a bar coating method, a die coating method, a reverse coating method, a comma coating method, a gravure coating method, and a doctor knife method, can be applied. Among these, it is preferable to use an immersion method (a dipping method, a dip coating method).

Incidentally, in a case where a lubricating layer is formed on a fine and narrow inner surface of a catheter, a guide wire, an injection needle, or the like, the base layer may be immersed in the coating liquid and then defoamed by reducing the pressure inside the system. By defoaming under reduced pressure, the solution can be caused to rapidly penetrate into the fine and narrow inner surface, and the formation of a lubricating layer can be promoted.

Furthermore, in a case where the lubricating layer is formed only on a portion of the base layer, only the portion of the base layer is immersed in the coating liquid to coat the portion of the base layer with the coating liquid, and thereby a lubricating layer can be formed at a desired surface site of the base layer.

In a case where it is difficult to immerse only a portion of the base layer in the coating liquid, the surface portion of the base layer where it is not necessary to form a lubricating layer is protected (covered, for example) in advance with an appropriate member or material that is attachable and detachable (demountable or mountable), subsequently the base layer is immersed in the coating liquid to coat the base layer with the coating liquid, subsequently the protective member (material) at the surface portion of the base layer where it is not necessary to form a lubricating layer is eliminated, subsequently the surface part is caused to react by a heating treatment or the like, and thereby a lubricating layer can be formed at a desired surface site of the base layer. However, in this invention, there is no limitation on such a method of forming, and a lubricating layer can be formed by appropriately utilizing any conventionally known method. For example, in a case where it is difficult to immerse only a portion of the base layer in the coating liquid, other coating techniques (for example, a method of applying a coating liquid on a predetermined surface portion of a medical instrument using an application apparatus such as a spray apparatus, a bar coater, a die coater, a reverse coater, a comma coater, a gravure coater, a spray coater, or a doctor knife) may also be applied instead of an immersion method. Incidentally, in a case where both an outer surface and an inner surface of a cylindrical instrument need to have a lubricating layer due to the structure of a medical instrument, an immersion method (dipping method) is preferably used from the viewpoint that both the outer surface and the inner surface can be coated at one time.

A coating amount of the block copolymer solution (coating liquid) is preferably an amount with which a thickness of a coating film (lubricating layer) to be obtained becomes 0.1 to 10 µm, more preferably an amount with which the thickness becomes 0.5 to 5 µm, and even more preferably an amount with which the thickness becomes 1 to 3 µm. When the coating amount is an amount with which the thickness of the coating film (lubricating layer) becomes 0.1 µm or more, durability of the coating film (lubricating layer) to be obtained can be sufficiently achieved. Furthermore, when the coating amount is an amount with which the thickness of the coating film (lubricating layer) becomes 10 µm or less, the surface of the coating film (lubricating layer) is less likely to become sticky, and handling during manufacture becomes easier.

### (II) Drying and/or heating treatment step

In the method for manufacturing a medical instrument according to the invention, it is preferable to carry out a drying and/or heating treatment step after a coating layer is formed by applying the block copolymer solution (coating liquid) on the base layer, for the purpose of removing the solvent and forming a firm lubricating layer.

In the following description, with regard to the (II) drying and/or heating treatment step of the block copolymer solution (coating liquid), a preferred embodiment will be described in detail.

After a solution (coating liquid) including a block copolymer and a choline derivative is applied on a base layer in the (I) solution application step, it is preferable to carry out a drying and/or heating treatment. Here, the terms "drying treatment" and "heating treatment" are not to be strictly distinguished; however, for the convenience of explanation, the "drying treatment" means that the base layer having the coating liquid applied thereon is maintained at or below a temperature near room temperature (20°C to 30°C), and the "heating treatment" means that the base layer is maintained at a temperature higher than a temperature near room temperature (20°C to 30°C).

The conditions at the time of the drying or heating treatment are not particularly limited as long as they are conditions in which a lubricating layer including a block copolymer can be formed on the base layer.

The temperature for the drying or heating treatment is not particularly limited; however, the temperature is preferably 10°C to 200°C. That is, it is more preferable that after the block copolymer solution is applied on the base layer (after a coating layer is formed), the coating layer is maintained at 10°C to 200°C. By maintaining the coating layer at such a temperature, cross-linking or polymerization of the block copolymer is effectively promoted, and a firm coating layer (lubricating layer) is formed. Therefore, a high lubricating property (surface lubricating property) can be maintained over a longer period of time. Furthermore, by maintaining the coating layer at such a temperature, it is possible to suppress excessive proceeding of the cross-linking or polymerization. Accordingly, it is possible to suppress deterioration in a swelling property attributable to the lubricating layer becoming too hard, and as a result, a favorable lubricating property (surface lubricating property) can be maintained.

In addition, after the block copolymer solution is applied on the base layer (after the coating layer is formed), it is more preferable that the coating layer is maintained at 110°C or lower, even more preferably at 25°C to 110°C, and most preferably at 45°C to 100°C. When the coating layer is maintained at such a temperature, a lubricating layer that can exhibit an excellent lubricating property and has high durability can be formed. Particularly, by maintaining the temperature at 110°C or lower, excessive cross-linking or polymerization of the block copolymer can be suppressed even when the heating treatment is performed for a long period of time. Accordingly, it is possible to suppress deterioration in a swelling property attributable to the lubricating layer becoming too hard, and the lubricating property can be controlled more easily. Incidentally, the above-described temperature may be changed in the middle of the drying or heating treatment.

In the manufacturing method according to the invention, it is also one of the features that a lubricating layer having high durability can be formed even when the block copolymer solution (coating liquid) is applied as described above and then maintained at a relatively low temperature of, for example, 80°C or lower, or 25°C to 80°C. As a result, there is an advantage that even a polymer material that is easily deformed or plasticized by heat can be used as the base layer. Therefore, according to the invention, the selectivity for the material is increased, and medical instruments for various use applications can be manufactured. Furthermore, since a lubricating layer having excellent durability can be formed at low temperatures, it is also preferable from the energy cost at the time of manufacturing medical instruments.

A time for the drying or heating treatment is also not particularly limited; however, the time is preferably 30 minutes to 30 hours, more preferably 1 to 25 hours, and particularly preferably 1 to 10 hours. By adopting such a time, cross-linking or polymerization within the block copolymer is effectively promoted, and a firm coating layer (lubricating layer) is formed. Accordingly, a high lubricating property (surface lubricating property) can be maintained over a longer period of time. Furthermore, by adopting such a time, it is possible to suppress excessive proceeding of the cross-linking or polymerization. Therefore, it is possible to suppress deterioration in a swelling property attributable to the lubricating layer becoming too hard, and as a result, a favorable lubricating property (surface lubricating property) can be maintained.

According to the present step, from the viewpoint of especially effectively (efficiently) promoting cross-linking or polymerization of the block copolymer, it is preferable to perform a drying treatment and then further perform a heating treatment. As such, since a heating treatment is further carried out in a state in which the solvent has been distilled off (that is, in a state in which the block copolymer and the choline derivative can be easily brought into contact with each other) by performing the drying and heating treatments, the effect of the choline derivative for promoting cross-linking or polymerization of the block copolymer is further enhanced. Accordingly, since a heating treatment can be carried out in a shorter time, even a polymer material that is easily deformed or plasticized by heat can be used as the base layer.

The conditions for the drying and heating treatment (temperature, time, and the like) at this time are also not particularly limited; however, from the viewpoint of efficiently manufacturing a medical instrument, it is preferable to perform a drying treatment in which the temperature is maintained for 1 minute to 5 hours at 10°C to 30°C and then to perform a heating treatment in which the temperature is maintained for 1 to 15 hours at 40°C to 200°C. Furthermore, from a similar point of view, it is more preferable to perform a drying treatment in which the temperature is maintained for 1 minute to 3 hours at 20°C to 30°C and then to perform a heating treatment in which the temperature is maintained for 2 to 12 hours at 45°C to 150°C, and it is particularly preferable to perform a drying treatment in which the temperature is maintained for 1 minute to 1.5 hours at 20°C to 25°C and then to perform a heating treatment in which the temperature is maintained for 3 to 10 hours at 45°C to 100°C. As such, according to the method of the invention, a medical instrument comprising a lubricating layer having excellent durability can be manufactured at a low temperature and for a short period of time.

On the other hand, in a case where cross-linking or polymerization of the block copolymer is sufficiently promoted by the choline derivative included in the block copolymer solution, only a drying treatment may be performed without requiring a heating treatment. At this time, the conditions for the drying treatment (temperature, time, and the like) are not particularly limited; however, from the viewpoint that a lubricating layer having an excellent lubricating property and high durability is formed, the treatment temperature is preferably 10°C to 30°C, and more preferably 20°C to 25°C. Furthermore, the treatment time is preferably 5 to 30 hours, and more preferably 10 to 25 hours.

Thus, with regard to the drying and/or heating treatment, it is preferable to perform a drying treatment in which the temperature is maintained for 1 minute to 1.5 hours at 20°C to 25°C and then to perform a heating treatment in which the temperature is maintained for 3 to 10 hours at 45°C to 100°C, or to perform a drying treatment in which the temperature is maintained for 10 to 25 hours at 20°C to 25°C.

When conditions such as described above (temperature, time, and the like) are employed, a firm lubricating layer (coating layer) can be carried on the base layer surface. Furthermore, in a case where a functional group that can react with an epoxy group (for example, a carboxyl group, an amino group, or a hydroxyl group) is present at the base layer surface, the functional group on the base layer surface and an epoxy group in the block copolymer within the lubricating layer are subjected to a cross-linking reaction, and covalent bonds are formed between the base layer and the lubricating layer. Therefore, a lubricating layer having high strength, which will not be easily detached from the base layer, can be formed. Accordingly, detachment of the lubricating layer from the base layer can be effectively suppressed and prevented by the drying and/or heating treatment step.

Furthermore, the pressure conditions during drying are also not limited, and drying may be performed under pressure or under reduced pressure, in addition to performing at normal pressure (atmospheric pressure).

Regarding drying or heating means (apparatus), for example, an oven, a reduced pressure dryer, or the like can be utilized; however, in the case of natural drying, drying means (apparatus) in particular is unnecessary.

### (III) Washing step

In the method for manufacturing a medical instrument according to the invention, after the (I) solution application step or the (II) drying and/or heating treatment step that is optionally carried out, the lubricating layer provided on the base layer is washed as necessary ((III) washing step) . A washing step is carried out for the purpose of removing the choline derivative included in the block copolymer solution (coating liquid). That is, according to a preferred embodiment of the invention, after the coating liquid is applied on the base layer to form a coating film (lubricating layer), the coating film (lubricating layer) is washed.

Hereinafter, a preferred embodiment of the (III) washing step will be described in detail.

A washing method is not particularly limited; however, a method of immersing a coating film (lubricating layer) in a washing solvent, a method of causing a washing solvent to flow over a coating film (lubricating layer), or a combination of these may be used. The washing solvent used at this time is not particularly limited as long as the washing solvent does not dissolve the coating film (lubricating layer) formed by the block copolymer and can remove impurities including the choline derivative; however, water or warm water is preferably used. A temperature of washing water is not particularly limited; however, the temperature is preferably 20°C to 100°C, and more preferably 25°C to 80°C. Furthermore, a washing time (time for bringing the washing solvent into contact with the coating film) is not particularly limited; however, the washing time is preferably 1 to 60 minutes, and more preferably 5 to 30 minutes. According to the conditions described above, the choline derivative can be sufficiently removed. Even after washing, the lubricating layer formed on the base layer can exhibit an excellent lubricating property.

After the washing step, a drying step may be further carried out. A drying method and drying conditions (temperature, time, and the like) are not particularly limited, and any conventionally known method can be used.

A medical instrument manufactured by the method according to the invention has a configuration in which a lubricating layer is carried on a base layer, by applying a solution (coating liquid) including a block copolymer that forms the lubricating layer and a choline derivative on the base layer. At this time, from the viewpoint of a lubricating property, the choline derivative included in the lubricating layer may be completely removed by washing or may remain without being completely removed. In such a case, it is determined that the medical instrument has been manufactured by the method according to the invention. The presence of the choline derivative in the lubricating layer is checked specifically by the fact that the choline derivative is detected by the following known analysis methods such as ATR-IR and LC-MS.

ATR-IR is performed under, for example, the following conditions.

### <<ATR-IR analysis conditions>>

Apparatus: Fourier transform infrared spectrophotometer Spectrum 100 manufactured by PerkinElmer, Inc.
Measurement mode: ATR method
Detector: ZnSe
Resolution: 4 cm⁻¹
Measuring range: 4000 to 650 cm⁻¹
Number of times of integration: 4 times

In the case of performing a washing step, an amount of the choline derivative included in the lubricating layer after the washing step is preferably 50 µg/cm² or less per unit area, and may also be 5 µg/cm² or less. On the other hand, the lower limit is not particularly limited; however, when a substantial removal efficiency of the choline derivative and a practical lubricating property of the lubricating layer are taken into consideration, the lower limit is 1 µg/cm² or more.

Here, a content of the choline derivative "per unit area" is defined as a value obtained by dividing a mass (µg) of the choline derivative included per unit area (1 cm²) of the lubricating layer, the choline derivative being measured by the following method, by an area (cm²) of the lubricating layer.

Specifically, a lubricating layer (medical instrument provided with a lubricating layer) is immersed in water and maintained for 24 hours at 70°C. Then, an extract (water) thus obtained is subjected to LC-MS measurement under the following conditions.

### <<LC/MS measurement conditions>>

LC/MS instrument: Waters 2695/Quattro microAPI (LC)
LC model: Waters 2695
Column: XBridge Amide manufactured by Waters Corp. 2.1 mm × 15 mm, 3.5 µm
Mobile phase: 20 mmol/L solution of ammonium formate (pH 3.2)/acetonitrile (1 : 9 (volume ratio))
Flow rate: 0.2 mL/min
Column temperature: 40°C
(MS)
MS model: Waters Quattro micro API
Ionization method: ESI-Positive

### <Medical instrument>

A medical instrument having a lubricating layer (coating layer) exhibiting excellent durability can be manufactured by carrying out the (I) solution application step (coating layer forming step) described above and the (II) drying and/or heating treatment step and (III) washing step, which are carried out as necessary.

That is, according to the method according to the invention, a firm lubricating layer that will not be easily detached from the base layer can be formed by forming an application layer including a block copolymer and a choline derivative on a surface of a base layer and then crosslinking epoxy groups. Furthermore, the medical instrument obtainable by the method according to the invention can exhibit excellent lubricating property and lubrication retaining property (sliding durability) because a lubricating layer formed by a block copolymer is formed on the surface of the instrument. At this time, even when the choline derivative remains in the lubricating layer, favorable sliding durability can be exhibited.

Accordingly, the invention also provides a medical instrument comprising a base layer and a lubricating layer carried on at least a portion of the base layer, in which the lubricating layer includes a block copolymer having a structural unit (A) derived from a reactive monomer having an epoxy group and a structural unit (B) derived from a hydrophilic monomer, and a choline derivative, wherein the molar ratio between the structural unit (A) and the structural unit (B) of the block copolymer ranges from 1:10 to 1:30 and the choline derivative is at least one selected from the group consisting of a compound represented by Formula 1, as defined in independent claim 7, and a polymer having a structural unit represented by Formula 2, as defined in independent claim 7.

With regard to the medical instrument according to an exemplary embodiment of the invention, preferred embodiments of the base layer, the block copolymer, the choline derivative, and the solvent are similar to those described in the section <Method for manufacturing medical instrument>, and therefore, further explanation will not be repeated here.

It is preferable that the lubricating layer in the medical instrument of the invention includes the block copolymer and the choline derivative at a mass ratio of more than 1 : 0 and 1 : 1 or less, and more preferably at a mass ratio of 1 : 0.005 to 1 : 1. The mass ratio of the block copolymer and the choline derivative in the lubricating layer can be checked by known analysis methods such as ATR-IR and LC-MS. Furthermore, in a case where a washing step of the lubricating layer is not carried out, the mass ratio of the block copolymer and the choline derivative in the lubricating layer corresponds to a mass ratio of the block copolymer and the choline derivative in a coating liquid for forming the lubricating layer.

Hereinafter, a preferred exemplary embodiment of a medical instrument that is manufactured by the method according to the invention will be described with reference to the attached drawings.

Fig. 1 is a partial cross-sectional view schematically showing a laminated structure of a surface of a representative exemplary embodiment of a medical instrument manufactured by the method according to the invention (in the present specification, also simply described as "medical instrument"). Fig. 2 is a partial cross-sectional view schematically showing a configuration example in which a laminated structure of the surface is different, as an application example of the present exemplary embodiment. Incidentally, various reference numerals in Fig. 1 and Fig. 2 respectively represent the following. Reference numeral 1 denotes a base layer; reference numeral 1a denotes a base layer core part; reference numeral 1b denotes a base surface layer; reference numeral 2 denotes a lubricating layer; and reference numeral 10 denotes a medical instrument that is manufactured by the method according to the invention.

As shown in Fig. 1 and Fig. 2, the medical instrument 10 of the present exemplary embodiment comprises a base layer 1 and a lubricating layer 2 including a block copolymer, which is provided on at least a portion of the base layer 1 (in the figure, an example in which the lubricating layer is provided over the entirety of the surface (entire surface) of the base layer 1 in the figure is shown). Incidentally, in Fig. 1 and Fig. 2, the lubricating layer 2 is formed on both surfaces of the base layer 1; however, the invention is not limited to the embodiment described above and may be any embodiment such as an embodiment in which the lubricating layer 2 is formed on one surface of the base layer 1; or an embodiment in which the lubricating layer 2 is formed on a portion of one surface or both surfaces of the base layer 1.

Hereinafter, each configuration member of the medical instrument will be described in detail.

### <<Base layer (base)>>

The base layer that is used in the present exemplary embodiment may be formed from any material, and the material is not particularly limited. Specifically, examples of the material constituting the base layer 1 include metal materials, polymer materials, and ceramics. Incidentally, specific examples of the material that constitutes the base layer 1 are as described in the section <<Application of block copolymer solution (coating liquid)>>.

Here, the base layer 1 may be such that the entire base layer 1 is formed of any of the above-described materials. The base layer 1 may have a multilayer structure obtained by laminating different materials into multiple layers, a structure obtained by connecting members formed from different materials for different parts of a medical instrument, or the like. Alternatively, as shown in Fig. 2, the base layer 1 may have a structure in which a base surface layer 1b is formed by coating a surface of a base layer core part 1a formed from any of the above-described materials with any of the above-described materials by an appropriate method. Examples of the latter case include a case where a base surface layer 1b is formed by coating a surface of a base layer core part 1a formed from a resin material or the like with a metal material by an appropriate method (conventionally known method such as plating, metal vapor deposition, or sputtering) ; and a case where a base surface layer 1b is formed by coating a surface of a base layer core part 1a formed from a hard reinforcing material such as a metal material or a ceramic material, with a polymer material that is flexible compared to the reinforcing material such as a metal material by an appropriate method (conventionally known method such as immersion (dipping), spraying (spray), or application and printing), or by compositizing the reinforcing material that forms the base layer core part 1a with the polymer material. Furthermore, the base layer core part 1a may be a multilayer structure obtained by laminating different materials into multiple layers, a structure obtained by connecting members formed from different materials for each portion of the medical instrument, or the like. Furthermore, a separate middle layer (not shown in the figure) may be further formed between the base layer core part 1a and the base surface layer 1b. In addition, the base surface layer 1b may also be a multilayer structure obtained by laminating different materials into multiple layers, a structure obtained by connecting members formed from different materials for each portion of the medical instrument, or the like.

### <<Lubricating layer (surface lubricating layer, coating layer)>>

A lubricating layer is carried on at least a portion of the base layer 1. Here, it is said that the lubricating layer 2 is carried on at least a portion of a surface of the base layer 1, because for a medical instrument such as a catheter, a guide wire, or an indwelling needle, which are use applications, it is not necessarily essential that all surfaces (entire surface) of such a medical instrument has a lubricating property when wetted, and it is desirable that the lubricating layer is carried only on a surface portion (there is a case of a portion, and there is also a case of entirety) where the surface is required to have a lubricating property when wetted. Therefore, as described above, an embodiment in which the lubricating layer is formed so as to cover the entirety of both surfaces of the base layer as shown in Fig. 1 and Fig. 2; an embodiment in which the lubricating layer is formed so as to cover only the entirety of one surface of the base layer; an embodiment in which the lubricating layer is formed so as to cover a portion of both surfaces of the base layer in the same form or different forms; an embodiment in which the lubricating layer is formed so as to cover a portion of one surface of the base layer; and the like are included.

### <Use application of medical instrument>

The use of the medical instrument, as described here below, does not form part of the claimed invention. A medical instrument manufactured by the method of the invention is a device that is used by being brought into contact with a body fluid, blood, or the like, a surface of the device having a lubricating property in a water-based liquid such as a body fluid or physiological saline, and the medical instrument can bring an enhancement in maneuverability and reduction of damage to the tissue mucosa. Specific examples include a catheter, a guide wire, and an indwelling needle, all of which are used in blood vessels, and the following medical instruments may also be shown in addition thereto.
(a) Catheters that are orally or nasally inserted or indwelled in the digestive tract, such as a gastric tube catheter, a feeding catheter, and a tube for tubal feeding.
(b) Catheters that are orally or nasally inserted or indwelled in the respiratory tract or trachea, such as an oxygen catheter, an oxygen cannula, a tube and a cuff for an endotracheal tube, a tube and a cuff for a tracheostomy tube, and an endotracheal suction catheter.
(c) Catheters that are inserted or indwelled in the urethra or ureter, such as a urethral catheter, a urinary catheter, and a catheter and a balloon for a urethral balloon catheter.
(d) Catheters that are inserted or indwelled in various lumens in a living body, organs, and tissues, such as a suction catheter, a drainage catheter, and a rectal catheter.
(e) Catheters that are inserted or indwelled in blood vessels, such as an indwelling needle, an IVH catheter, a thermodilution catheter, a catheter for angiography, a catheter for vasodilation, and a dilator or an introducer, or a guide wire, a stylet, and the like for these catheters.
(f) Artificial trachea, artificial bronchi, and the like.
(g) Medical instruments for extracorporeal circulation treatment (an artificial lung, an artificial heart, an artificial kidney, and the like) and circuits thereof.

### Example

The effects of the invention will be described using the following Examples and Comparative Examples. However, the technical scope of the invention is not intended to be limited to the following Examples only. Incidentally, in the following Examples, unless particularly stated otherwise, operations were carried out at room temperature (25°C). Furthermore, unless particularly stated otherwise, units "%" and "parts" mean "% by mass" and "parts by mass", respectively.

### [Synthesis Example 1: Synthesis of block copolymer (1)]

The following reaction was carried out, and a block copolymer (1) was produced.

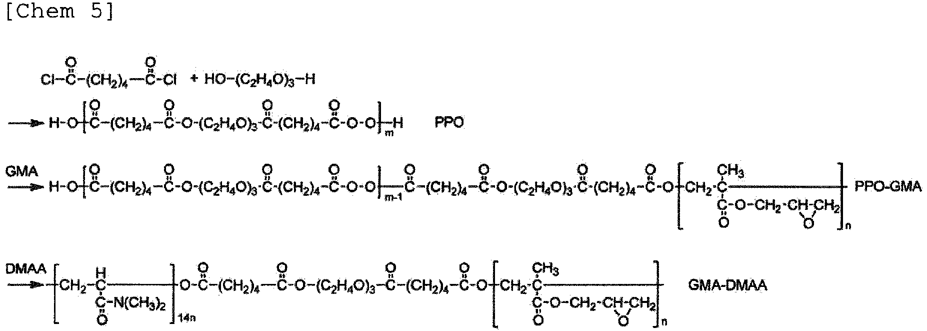

29.7 g of triethylene glycol was added dropwise to 72.3 g of adipic dichloride at 50°C, subsequently hydrochloric acid was removed under reduced pressure at 50°C for 3 hours, and thus an oligoester was obtained. Next, 4.5 g of methyl ethyl ketone was added to 22.5 g of the oligoester thus obtained, this was added dropwise into a solution comprising 5 g of sodium hydroxide, 6.93 g of 31% hydrogen peroxide, 0.44 g of dioctyl phosphate as a surfactant, and 120 g of water, and the mixture was caused to react for 20 minutes at -5°C. The product thus obtained was repeatedly washed with water and washed with methanol, subsequently the product was dried, and thereby a polyperoxide (PPO) having a plurality of peroxide groups in the molecule was obtained.

Next, 0.5 g of this PPO, 9.5 g of glycidyl methacrylate (GMA), and 30 g of benzene as a solvent were subjected to polymerization while being stirred under reduced pressure at 80°C for 2 hours. The reaction product obtained after polymerization was reprecipitated with diethyl ether, and thereby polyglycidyl methacrylate having a plurality of peroxide groups in the molecule (PPO-GMA) was obtained.

Subsequently, 1.0 g of PPO-GMA thus obtained (corresponding to 7 mmol of GMA) was introduced into 9.0 g of N,N-dimethylacrylamide (DMAA) and 90 g of dimethyl sulfoxide as a solvent, and the mixture was caused to react at 80°C for 18 hours. The reaction product obtained after the reaction was reprecipitated with hexane and collected, and a block copolymer (1) (structural unit (A) : structural unit (B) = GMA : DMAA = 1 : 14 (molar ratio)) having an epoxy group in the molecule and exhibiting a lubricating property when wetted was obtained. The block copolymer (1) obtained in this manner was analyzed by ¹H-NMR and ATR-IR, and it was confirmed that epoxy groups were present in the molecule. Furthermore, a weight average molecular weight (Mw) of the block copolymer (1) measured by gel permeation chromatography (GPC, calculated relative to polystyrene standards) was about 1,500,000.

### [Example 1]

The block copolymer (1) obtained in the Synthesis Example 1 described above was dissolved in N,N-dimethylformamide (DMF) such that a final concentration in a coating liquid would be 6% by mass, choline chloride (manufactured by Fujifilm Wako Pure Chemical Corp.) was added in an amount (mass ratio) 0.1 times the amount of the block copolymer (1) and dissolved, and a coating liquid (choline chloride concentration was 0.6% by mass) was prepared. A tube made of polyethylene (NOVATEC (registered trademark) HB530 manufactured by Japan Polyethylene Corp.) having an outer diameter of 0.84 mm was immersed in the coating liquid and dried at room temperature (25°C) for 1 hour to form a coating film. Furthermore, the coating film was heat-treated by storing the tube in an oven at 80°C for 5 hours. The tube was cooled to room temperature and then immersed in water at room temperature (25°C) for 10 minutes, and thereby choline chloride was eluted and removed from the coating film. The tube was dried at room temperature (25°C), and a coated tube (1) having a coating layer (lubricating layer) (film thickness after drying = 2 µm) including a cross-linked copolymer derived from the block copolymer (1) on the surface was produced.

This coated tube (1) was subjected to a surface analysis by ATR-IR, a peak for an epoxy group became small, and a reaction of an epoxy group was confirmed. Furthermore, a peak for choline chloride was not recognized.

This coated tube (1) was immersed in physiological saline at 25°C and rubbed with a finger, and it was confirmed that a slippery low-friction surface was formed as compared to an untreated tube.

Incidentally, the surface analysis by ATR-IR was carried out under the following conditions (hereinafter, the same applies).

### <<ATR-IR analysis conditions>>

Apparatus: Fourier transform infrared spectrophotometer Spectrum 100 manufactured by PerkinElmer, Inc.
Measurement mode: ATR method
Detector: ZnSe
Resolution: 4 cm⁻¹
Measuring range: 4000 to 650 cm⁻¹
Number of times of integration: 4 times

### [Example 2]

A coated tube (2) was produced by a method similar to that of Example 1, except that a coating liquid was prepared by adding choline bromide (manufactured by Tokyo Chemical Industry Co., Ltd.) instead of choline chloride, in an amount (mass ratio) 0.1 times the amount of the block copolymer (1).

This coated tube (2) was subjected to a surface analysis by ATR-IR, a peak for an epoxy group became small, and a reaction of an epoxy group was confirmed. Furthermore, a peak for choline bromide was not recognized.

This coated tube (2) was immersed in physiological saline at 25°C and rubbed with a finger, and it was confirmed that a slippery low-friction surface was formed as compared to an untreated tube.

### [Example 3]

A coated tube (3) was produced by a method similar to that of Example 1, except that a coating liquid was prepared by adding choline iodide (manufactured by Tokyo Chemical Industry Co., Ltd.) instead of choline chloride, in an amount (mass ratio) 0.1 times the amount of the block copolymer (1).

This coated tube (3) was subjected to a surface analysis by ATR-IR, a peak for an epoxy group became small, and a reaction of an epoxy group was confirmed. Furthermore, a peak for choline iodide was not recognized.

This coated tube (3) was immersed in physiological saline at 25°C and rubbed with a finger, and it was confirmed that a slippery low-friction surface was formed as compared to an untreated tube.

### [Example 4]

A coated tube (4) was produced by a method similar to that of Example 1, except that a coating liquid was prepared by using dimethyl sulfoxide (DMSO) instead of N,N-dimethylformamide (DMF), and adding choline dihydrogen citrate (manufactured by Tokyo Chemical Industry Co., Ltd.) instead of choline chloride, in an amount (mass ratio) 0.1 times the amount of the block copolymer (1).

This coated tube (4) was subjected to a surface analysis by ATR-IR, a peak for an epoxy group became small, and a reaction of an epoxy group was confirmed. Furthermore, a peak for choline dihydrogen citrate was not recognized.

This coated tube (4) was immersed in physiological saline at 25°C and rubbed with a finger, and it was confirmed that a slippery low-friction surface was formed as compared to an untreated tube.

### [Example 5]

A coated tube (5) was produced by a method similar to that of Example 1, except that a coating liquid was prepared by adding choline bitartrate (manufactured by Tokyo Chemical Industry Co., Ltd.) instead of choline chloride, in an amount (mass ratio) 0.1 times the amount of the block copolymer (1).

This coated tube (5) was subjected to a surface analysis by ATR-IR, a peak for an epoxy group became small, and a reaction of an epoxy group was confirmed. Furthermore, a peak for choline bitartrate was not recognized.

This coated tube (5) was immersed in physiological saline at 25°C and rubbed with a finger, and it was confirmed that a slippery low-friction surface was formed as compared to an untreated tube.

### [Example 6]

A coated tube (6) was produced by a method similar to that of Example 1, except that a coating liquid was prepared by adding acetylcholine chloride (manufactured by Tokyo Chemical Industry Co., Ltd.) instead of choline chloride, in an amount (mass ratio) 0.1 times the amount of the block copolymer (1).

This coated tube (6) was subjected to a surface analysis by ATR-IR, a peak for an epoxy group became small, and a reaction of an epoxy group was confirmed. Furthermore, a peak for acetylcholine chloride was not recognized.

This coated tube (6) was immersed in physiological saline at 25°C and rubbed with a finger, and it was confirmed that a slippery low-friction surface was formed as compared to an untreated tube.

### [Example 7]

A coated tube (7) was produced by a method similar to that of Example 1, except that a coating liquid was prepared by adding benzoylcholine chloride (manufactured by Tokyo Chemical Industry Co., Ltd.) instead of choline chloride, in an amount (mass ratio) 0.1 times the amount of the block copolymer (1).

This coated tube (7) was subjected to a surface analysis by ATR-IR, a peak for an epoxy group became small, and a reaction of an epoxy group was confirmed. Furthermore, a peak for benzoylcholine chloride was not recognized.

This coated tube (7) was immersed in physiological saline at 25°C and rubbed with a finger, and it was confirmed that a slippery low-friction surface was formed as compared to an untreated tube.

### [Example 8]

A coated tube (8) was produced by a method similar to that of Example 1, except that a coating liquid was prepared by using dimethyl sulfoxide (DMSO) instead of N,N-dimethylformamide (DMF) and adding acryloylcholine chloride (manufactured by Tokyo Chemical Industry Co., Ltd.) instead of choline chloride, in an amount (mass ratio) 0.1 times the amount of the block copolymer (1).

This coated tube (8) was subjected to a surface analysis by ATR-IR, a peak for an epoxy group became small, and a reaction of an epoxy group was confirmed. Furthermore, a peak for acryloylcholine chloride was not recognized.

This coated tube (8) was immersed in physiological saline at 25°C and rubbed with a finger, and it was confirmed that a slippery low-friction surface was formed as compared to an untreated tube.

### [Example 9]

1.9 g of a 80 wt% aqueous solution of acryloylcholine chloride (manufactured by Sigma-Aldrich Corp.) was dissolved in 4 g of ethanol, nitrogen gas was introduced into the liquid for several seconds, 3 mg of a polymerization initiator, V-70 (manufactured by Fujifilm Wako Pure Chemical Corp., 2,2'-azobis(4-methoxy-2,4-dimethylvaleronitrile)) was added thereto, and the mixture was caused to react at 50°C for 5 hours. A reaction product obtained after the reaction was reprecipitated with hexane and collected, the reaction product was dried under reduced pressure at room temperature for 5 hours, and poly (acryloylcholine chloride) was obtained. The polymer was analyzed by ¹H-NMR and ATR-IR, and it was confirmed that the polymer was a homopolymer of acryloylcholine chloride. Furthermore, a weight average molecular weight (Mw) of poly(acryloylcholine chloride) measured by gel permeation chromatography (GPC, calculated relative to polystyrene standards) was about 200,000.

A coated tube (9) was produced by a method similar to that of Example 1, except that a coating liquid was prepared by using dimethyl sulfoxide (DMSO) instead of N,N-dimethylformamide (DMF) and adding the poly(acryloylcholine chloride) synthesized as described above instead of choline chloride, in an amount (mass ratio) 0.1 times the amount of the block copolymer (1).

This coated tube (9) was subjected to a surface analysis by ATR-IR, a peak for an epoxy group became small, and a reaction of an epoxy group was confirmed. Furthermore, a peak for poly(acryloylcholine chloride) was not recognized.

This coated tube (9) was immersed in physiological saline at 25°C and rubbed with a finger, and it was confirmed that a slippery low-friction surface was formed as compared to an untreated tube.

### [Example 10]

1.4 g of dimethylacrylamide (DMAA) and 0.2 g of a 80 wt% aqueous solution of acryloylcholine chloride (manufactured by Sigma-Aldrich Corp.) were dissolved in 4 g of ethanol, nitrogen gas was introduced into the liquid for several seconds, 3 mg of a polymerization initiator, V-70 (manufactured by Fujifilm Wako Pure Chemical Corp.) was added thereto, and the mixture was caused to react at 50°C for 5 hours. A reaction product obtained after the reaction was reprecipitated with hexane and collected, the reaction product was dried under reduced pressure at room temperature for 5 hours, and poly(DMAA-acryloylcholine chloride) (DMAA : acryloylcholine chloride = 18 : 1 (molar ratio)) was obtained. The polymer was analyzed by ¹H-NMR and ATR-IR, and it was confirmed that the polymer was a copolymer of DMAA and acryloylcholine chloride. Furthermore, a weight average molecular weight (Mw) of poly (DMAA-acryloylcholine chloride) measured by gel permeation chromatography (GPC, calculated relative to polystyrene standards) was about 200,000.

A coated tube (10) was produced by a method similar to that of Example 1, except that a coating liquid was prepared by adding the poly(DMAA-acryloylcholine chloride) synthesized as described above instead of choline chloride, in an amount (mass ratio) 0.1 times the amount of the block copolymer (1).

This coated tube (10) was subjected to a surface analysis by ATR-IR, a peak for an epoxy group became small, and a reaction of an epoxy group was confirmed. Furthermore, a peak for poly(DMAA-acryloylcholine chloride) was not recognized.

This coated tube (10) was immersed in physiological saline at 25°C and rubbed with a finger, and it was confirmed that a slippery low-friction surface was formed as compared to an untreated tube.

### [Example 11]

A coated tube (11) was produced by a method similar to that of Example 1, except that a coating liquid (choline chloride concentration was 0.03% by mass) was prepared by adding choline chloride in an amount (mass ratio) 0.005 times the amount of the block copolymer (1).

This coated tube (11) was subjected to a surface analysis by ATR-IR, a peak for an epoxy group became small, and a reaction of an epoxy group was confirmed. Furthermore, a peak for choline chloride was not recognized

This coated tube (11) was immersed in physiological saline at 25°C and rubbed with a finger, and it was confirmed that a slippery low-friction surface was formed as compared to an untreated tube.

### [Example 12]

A coated tube (12) was produced by a method similar to that of Example 1, except that a coating liquid (choline chloride concentration was 0.03% by mass) was prepared by using dimethyl sulfoxide (DMSO) instead of N,N-dimethylformamide (DMF) and adding choline chloride in an amount (mass ratio) 0.005 times the amount of the block copolymer (1).

This coated tube (12) was subjected to a surface analysis by ATR-IR, a peak for an epoxy group became small, and a reaction of an epoxy group was confirmed. Furthermore, a peak for choline chloride was not recognized.

This coated tube (12) was immersed in physiological saline at 25°C and rubbed with a finger, and it was confirmed that a slippery low-friction surface was formed as compared to an untreated tube.

### [Example 13]

A coated tube (13) was produced by a method similar to that of Example 1, except that a coating liquid (choline chloride concentration was 0.6% by mass) was prepared by using dimethyl sulfoxide (DMSO) instead of N,N-dimethylformamide (DMF) and adding choline chloride in an amount (mass ratio) 0.1 times the amount of the block copolymer (1).

This coated tube (13) was subjected to a surface analysis by ATR-IR, a peak for an epoxy group became small, and a reaction of an epoxy group was confirmed. Furthermore, a peak for choline chloride was not recognized.

This coated tube (13) was immersed in physiological saline at 25°C and rubbed with a finger, and it was confirmed that a slippery low-friction surface was formed as compared to an untreated tube.

### [Example 14]

A coated tube (14) was produced by a method similar to that of Example 1, except that a coating liquid (choline chloride concentration was 6% by mass) was prepared by using dimethyl sulfoxide (DMSO) instead of N,N-dimethylformamide (DMF) and adding choline chloride in an amount (mass ratio) 1 time the amount of the block copolymer (1).

This coated tube (14) was subjected to a surface analysis by ATR-IR, a peak for an epoxy group became small, and a reaction of an epoxy group was confirmed. Furthermore, a peak for choline chloride was not recognized.

This coated tube (14) was immersed in physiological saline at 25°C and rubbed with a finger, and it was confirmed that a slippery low-friction surface was formed as compared to an untreated tube.

### [Example 15]

A coated tube (15) was produced by a method similar to that of Example 1, except that a coating liquid was prepared by using dimethyl sulfoxide (DMSO) instead of N,N-dimethylformamide (DMF), a tube made of a polyamide elastomer (VESTAMID (registered trademark) E62-S1 manufactured by Daicel-Evonik, Ltd.) instead of polyethylene was used, and the coating film was heat-treated by storing the tube in an oven at 50°C for 10 hours instead of storing in an oven at 80°C for 5 hours.

This coated tube (15) was subjected to a surface analysis by ATR-IR, a peak for an epoxy group became small, and a reaction of an epoxy group was confirmed. Furthermore, a peak for choline chloride was not recognized.

This coated tube (15) was immersed in physiological saline at 25°C and rubbed with a finger, and it was confirmed that a slippery low-friction surface was formed as compared to an untreated tube.

### [Example 16]

The block copolymer (1) obtained in Synthesis Example 1 was dissolved in N,N-dimethylformamide (DMF) such that a final concentration in the coating liquid would be 6% by mass, choline chloride (manufactured by Fujifilm Wako Pure Chemical Corp.) was added to this solution in an amount (mass ratio) 0.1 times the amount of the block copolymer (1) and dissolved therein, and a coating liquid (choline chloride concentration was 0.6% by mass) was prepared. A tube made of polyethylene (NOVATEC (registered trademark) HB530 manufactured by Japan Polyethylene Corp.) having an outer diameter of 0.84 mm was immersed in the coating liquid and dried at room temperature (25°C) for 1 hour to form a coating film. Furthermore, the coating film was heat-treated by storing the tube in an oven at 80°C for 5 hours, and a coated tube (16) having a coating layer (lubricating layer) (film thickness after drying = 2 µm) including a cross-linked copolymer derived from the block copolymer (1) and choline chloride on the surface was produced.

This coated tube (16) was subjected to a surface analysis by ATR-IR, a peak for an epoxy group became small, and a reaction of an epoxy group was confirmed. Furthermore, a peak for choline chloride was also recognized.

This coated tube (16) was immersed in physiological saline at 25°C and rubbed with a finger, and it was confirmed that a slippery low-friction surface was formed as compared to an untreated tube.

### [Comparative Example 1]

A comparative coated tube (1) was produced by a method similar to that of Example 1, except that a coating liquid was prepared without adding choline chloride.

This comparative coated tube (1) was immersed in physiological saline at 25°C and rubbed with a finger, and it was confirmed that a slippery low-friction surface was formed as compared to an untreated tube.

### [Comparative Example 2]

A comparative coated tube (2) was produced by a method similar to that of Example 1, except that a coating liquid (choline concentration was 0.6% by mass) was prepared by using dimethyl sulfoxide (DMSO) instead of N,N-dimethylformamide (DMF), using a 50% aqueous solution of choline (manufactured by Tokyo Chemical Industry Co., Ltd.) instead of choline chloride, and adding choline in an amount (mass ratio) 0.1 times the amount of the block copolymer (1).

This comparative coated tube (2) was immersed in physiological saline at 25°C and rubbed with a finger, and it was confirmed that a slippery low-friction surface was formed as compared to an untreated tube.

### [Evaluation of sliding durability]

For the coated tubes and comparative coated tubes obtained in Examples 1 to 16 and Comparative Examples 1 and 2 (hereinafter, also simply referred to as "samples"), sliding durability (durability of the lubricating layer) was evaluated by the following method.

### (Evaluation method)

Sliding property was subjected to sensory evaluation by a technique of immersing each sample in distilled water, putting the sample between hands, and rubbing the sample in a longitudinal direction. Sliding durability was evaluated by measuring the number of times of rubbing until a lubricating property (slippery sensation) was impaired. As the number of times is larger, it is considered that sliding durability is superior.

Results are shown in the following Table 1. For example, the description "> 10" implies that a sliding property was not impaired even when the sample was rubbed at least 10 times.

### [Table 1]

**Table 1**

| | Base | Choline derivative | | Solvent | Conditions for drying or heating treatment | Washing | Sliding durability |
|---|---|---|---|---|---|---|---|
| | | Type | Mass ratio (with respect to copolymer (1)) | | | | |
| Example 1 | Polyethylene | Choline chloride | 0.1 | DMF | 80°C × 5 hr | Present | > 50 |
| Example 2 | Polyethylene | Choline bromide | 0.1 | DMF | 80°C × 5 hr | Present | > 30 |
| Example 3 | Polyethylene | Choline iodide | 0.1 | DMF | 80°C × 5 hr | Present | > 30 |
| Example 4 | Polyethylene | Choline dihydrogen citrate | 0.1 | DMSO | 80°C × 5 hr | Present | > 20 |
| Example 5 | Polyethylene | Choline bitartrate | 0.1 | DMF | 80°C × 5 hr | Present | > 10 |
| Example 6 | Polyethylene | Acetylcholine chloride | 0.1 | DMF | 80°C × 5 hr | Present | > 30 |
| Example 7 | Polyethylene | Benzoylcholine chloride | 0.1 | DMF | 80°C × 5 hr | Present | > 30 |
| Example 8 | Polyethylene | Acryloylcholine chloride | 0.1 | DMSO | 80°C × 5 hr | Present | > 30 |
| Example 9 | Polyethylene | Polyacryloylcholine chloride | 0.1 | DMSO | 80°C × 5 hr | Present | > 30 |
| Example 10 | Polyethylene | Poly (DMAA-acryloylcholine chloride) | 0.1 | DMF | 80°C × 5 hr | Present | > 30 |
| Example 11 | Polyethylene | Choline chloride | 0.005 | DMF | 80°C × 5 hr | Present | > 30 |
| Example 12 | Polyethylene | Choline chloride | 0.005 | DMSO | 80°C × 5 hr | Present | > 30 |
| Example 13 | Polyethylene | Choline chloride | 0.1 | DMSO | 80°C × 5 hr | Present | > 50 |
| Example 14 | Polyethylene | Choline chloride | 1 | DMSO | 80°C × 5 hr | Present | > 30 |
| Example 15 | PA elastomer | Choline chloride | 0.1 | DMSO | 50°C × 10 hr | Present | > 30 |
| Example 16 | Polyethylene | Choline chloride | 0.1 | DMF | 80°C × 5 hr | Absent | > 50 |
| Comparative Example 1 | Polyethylene | None | - | DMF | 80°C × 5 hr | Present | 3 |
| Comparative Example 2 | Polyethylene | Choline | 0.1 | DMSO | 80°C × 5 hr | Present | 3 |

From Examples 1 to 16 of Table 1, it was found that a medical instrument having a lubricating layer having excellent sliding durability is obtained by applying a coating liquid including a block copolymer, a choline derivative, and a solvent on a base.

When a comparison of Examples 1 to 5 was made, in a case where a choline derivative in which X₁⁻ of the Formula (1) was a halide ion was used (Examples 1 to 3), excellent sliding durability was exhibited as compared to a case where a choline derivative in which X₁⁻ was an organic acid ion was used (Examples 4 and 5). Furthermore, when a comparison of Examples 1 to 3 was made, in a case where a choline derivative in which X₁⁻ was chloride ion was used (Example 1), excellent sliding durability was exhibited as compared to a case where a choline derivative in which X₁⁻ was bromide ion or iodide ion was used (Examples 2 and 3).

When a comparison of Examples 1 and 6 to 8 was made, in a case where a choline derivative in which R₁ of the Formula (1) was a hydrogen atom was used (Example 1), sliding durability was enhanced as compared to a case where a choline derivative in which R₁ was Rₐ-C(=O)- (definition of Rₐ is as described above) was used (Examples 6 to 8).

When a comparison of Examples 8 to 10 was made, in a case where a monomer of acryloylcholine chloride was used as the choline derivative (Example 8) and a case where a polymer of acryloylcholine chloride was used as the choline derivative (Examples 9 and 10), equal sliding durability was exhibited.

When a comparison of Examples 1 and 13 was made, it was found that the type of the solvent used in the coating liquid did not affect sliding durability. The same can be said also from a comparison of Examples 11 and 12.

When a comparison of Examples 12 to 14 was made, in a case where a mass ratio of the block copolymer and the choline derivative was 1 : 0.1 (Example 13), superior sliding durability was exhibited as compared to a case where the mass ratio was 1 : 0.005 or 1 : 1 (Examples 12 and 14).

When a comparison of Examples 1 and 16 was made, in a case where a washing operation was not performed after a lubricating layer was formed, and there was residual choline chloride in the lubricating layer (Example 16), sliding durability equal to that obtainable in a case where choline chloride was removed from the lubricating layer (Example 1) was exhibited.

Furthermore, according to the method of the invention, favorable sliding durability was obtained even when the heating treatment temperature after application was a low temperature, as in the case of Example 15. This is considered to be because a reaction of a glycidyl group in a copolymer sufficiently proceeds as a result of addition of a choline derivative.

On the other hand, in a case where a choline derivative was not used in the coating liquid, sliding durability was insufficient (Comparative Example 1). It is considered to be because cross-linking or polymerization of the block copolymer was not promoted, and a firm lubricating layer was not formed. Furthermore, even in a case where choline was used instead of a choline derivative, sliding durability was insufficient (Comparative Example 2). It is considered to be because, since choline is basic, cross-linking of the block copolymer was promoted in a stage of preparing a coating liquid, and the coating liquid could not be uniformly applied.

This application claims the benefit of Japanese Patent Application No. 2019-034808, filed on February 27, 2019.

### Reference Signs List

- 10: medical instrument
- 1: base layer
- 1a: base layer core part
- 1b: base surface layer
- 2: lubricating layer

## Claims

1. A method for manufacturing a medical instrument including a base layer and a lubricating layer carried on at least a portion of the base layer, the method comprising:
applying on the base layer a solution including: a block copolymer having a structural unit (A) derived from a reactive monomer having an epoxy group and a structural unit (B) derived from a hydrophilic monomer, a choline derivative, and a solvent,
wherein:
the molar ratio between the structural unit (A) and the structural unit (B) of the block copolymer ranges from 1:10 to 1:30, and
the choline derivative is at least one selected from the group consisting of a compound represented by the following Formula 1 and a polymer having a structural unit represented by the following Formula 2:
[Chem 1]
Formula 1 R₁-O-(CH₂)₂-N⁺(CH₃)₃ X₁⁻
wherein in Formula 1,
X₁⁻ represents a halide ion, an organic acid ion, or an inorganic acid ion; and
R₁ represents a hydrogen atom, a substituted or unsubstituted alkyl group having 1 to 12 carbon atoms, or Rₐ-C(=O)-, where Rₐ represents a substituted or unsubstituted alkyl group having 1 to 12 carbon atoms, a substituted or unsubstituted aryl group having 6 to 12 carbon atoms, CH₂=CH-, or CH₂=C(CH₃)-; 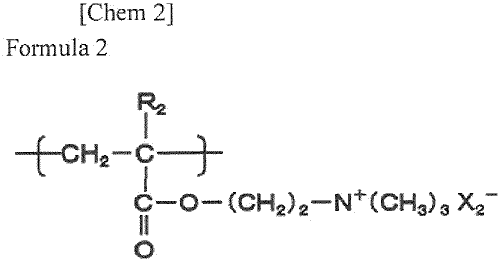
wherein in Formula 2,
X₂⁻ represents a halide ion, an organic acid ion, or an inorganic acid ion; and
R₂ represents a hydrogen atom or a methyl group.

2. The method for manufacturing a medical instrument according to claim 1, wherein the choline derivative is at least one selected from the group consisting of a compound represented by the following Formula 1 and a polymer having a structural unit represented by the following Formula 2:
[Chem 3]
Formula 1 R₁-O-(CH₂)₂-N⁺(CH₃)₃ X₁⁻
wherein in Formula 1,
X₁⁻ represents a halide ion or an organic acid ion; and
R₁ represents a hydrogen atom or RₐC(=O)-, where Rₐ represents a substituted or unsubstituted alkyl group having 1 to 12 carbon atoms, a substituted or unsubstituted aryl group having 6 to 12 carbon atoms, CH₂=CH-, or CH₂=C(CH₃)-; 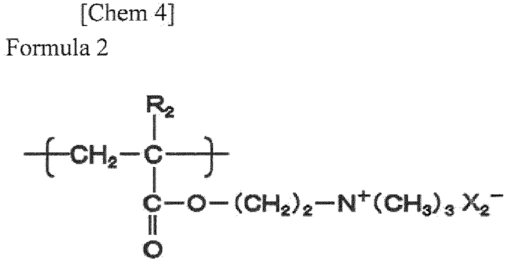 wherein in Formula 2,
X₂⁻ represents a halide ion or an organic acid ion; and
R₂ represents a hydrogen atom or a methyl group.

3. The method for manufacturing a medical instrument according to claim 1 or 2, wherein the reactive monomer having an epoxy group includes at least one selected from the group consisting of glycidyl acrylate, glycidyl methacrylate, 3,4-epoxycyclohexylmethyl acrylate, 3,4-epoxycyclohexylmethyl methacrylate, β-methylglycidyl methacrylate, and allyl glycidyl ether.

4. The method for manufacturing a medical instrument according to any one of claims 1 to 3, wherein the hydrophilic monomer includes at least one selected from the group consisting of N,N-dimethylacrylamide, acrylamide, 2-hydroxyethyl methacrylate, and N-vinylpyrrolidone.

5. The method for manufacturing a medical instrument according to any one of claims 1 to 4, wherein the solution includes 1% to 10% by mass of the block copolymer.

6. The method for manufacturing a medical instrument according to any one of claims 1 to 5, wherein the solution includes the block copolymer and the choline derivative at a mass ratio of 1 : 0.005 to 1 : 1.

7. A medical instrument comprising a base layer and a lubricating layer carried on at least a portion of the base layer,
the lubricating layer including a block copolymer having a structural unit (A) derived from a reactive monomer having an epoxy group and a structural unit (B) derived from a hydrophilic monomer, and a choline derivative,
wherein:
the molar ratio between the structural unit (A) and the structural unit (B) of the block copolymer ranges from 1:10 to 1:30, and
the choline derivative is at least one selected from the group consisting of a compound represented by the following Formula 1 and a polymer having a structural unit represented by the following Formula 2:
[Chem 5]
Formula 1 **R₁-O-(CH₂)₂-N⁺(CH₃)₃ X₁⁻**
wherein in Formula 1,
X₁⁻ represents a halide ion, an organic acid ion, or an inorganic acid ion; and
R₁ represents a hydrogen atom, a substituted or unsubstituted alkyl group having 1 to 12 carbon atoms, or Rₐ-C(=O)-, where Rₐ represents a substituted or unsubstituted alkyl group having 1 to 12 carbon atoms, a substituted or unsubstituted aryl group having 6 to 12 carbon atoms, CH₂=CH-, or CH₂=C(CH₃)-; 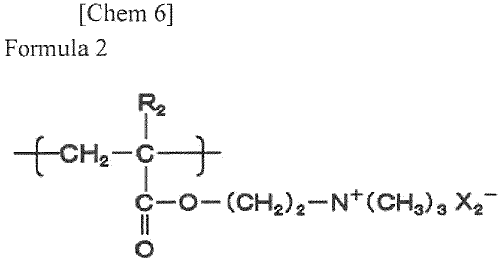
wherein in Formula 2,
X₂⁻ represents a halide ion, an organic acid ion, or an inorganic acid ion; and
R₂ represents a hydrogen atom or a methyl group.

8. The medical instrument according to claim 7, wherein the lubricating layer includes the block copolymer and the choline derivative at a mass ratio of 1 : 0.005 to 1 : 1.

## Patentansprüche

1. Verfahren zur Herstellung eines medizinischen Instruments, das eine Basisschicht und eine Gleitschicht umfasst, die auf mindestens einem Abschnitt der Basisschicht weitergeführt wird, wobei das Verfahren umfasst:
Aufbringen einer Lösung auf die Basisschicht, die umfasst: ein Blockcopolymer, das eine Struktureinheit (A) aufweist, die von einem reaktiven Monomer mit einer Epoxygruppe abgeleitet ist, und eine Struktureinheit (B), die von einem hydrophilen Monomer abgeleitet ist, ein Cholin-Derivat und ein Lösungsmittel, wobei:
das Molverhältnis zwischen der Struktureinheit (A) und der Struktureinheit (B) des Blockcopolymers in einem Bereich von 1:10 bis 1:30 liegt, und
das Cholin-Derivat mindestens eines ist, das aus der Gruppe ausgewählt ist, die aus einer Verbindung der folgenden Formel 1 und einem Polymer besteht, das eine Struktureinheit aufweist, die durch die folgende Formel 2 dargestellt wird:
[Chem 1]
Formel 1 **R₁-O-(CH₂)₂-N⁺(CH₃)₃ X₁⁻**
wobei in Formel 1,
X₁⁻ ein Halogenidion, ein Ion einer organischen Säure oder ein Ion einer anorganischen Säure darstellt; und
R₁ ein Wasserstoffatom, eine substituierte oder unsubstituierte Alkylgruppe mit 1 bis 12 Kohlenstoffatomen oder Rₐ-C(=O)- darstellt, wobei Rₐ eine substituierte oder unsubstituierte Alkylgruppe, die 1 bis 12 Kohlenstoffatome aufweist, eine substituierte oder unsubstituierte Arylgruppe, die 6 bis 12 Kohlenstoffatome aufweist, CH₂=CH- oder CH₂=C(CH₃)- darstellt; 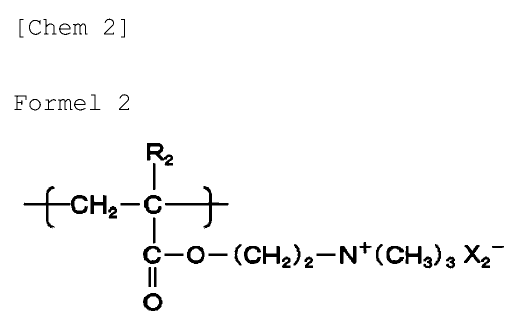
wobei in Formel 2,
X₂⁻ ein Halogenidion, ein Ion einer organischen Säure oder ein Ion einer anorganischen Säure darstellt; und
R₂ ein Wasserstoffatom oder eine Methylgruppe darstellt.

2. Verfahren zur Herstellung eines medizinischen Instruments nach Anspruch 1, wobei das Cholin-Derivat mindestens eines ist, das aus der Gruppe ausgewählt ist, die aus einer Verbindung der folgenden Formel 1 und einem Polymer besteht, das eine Struktureinheit aufweist, die durch die folgende Formel 2 dargestellt wird:
[Chem 3]
Formel 1 **R₁-Q- (CH₂)₂-N⁺(CH₃)₃ X₁⁻**
wobei in Formel 1,
X₁⁻ ein Halogenidion oder ein Ion einer organischen Säure darstellt; und
R₁ ein Wasserstoffatom oder Rₐ-C(=O)- darstellt, wobei Rₐ eine substituierte oder unsubstituierte Alkylgruppe, die 1 bis 12 Kohlenstoffatome aufweist, eine substituierte oder unsubstituierte Arylgruppe, die 6 bis 12 Kohlenstoffatome aufweist, CH₂=CH- oder CH₂=C(CH₃)- darstellt; 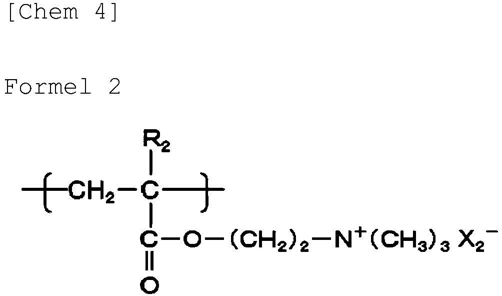
wobei in Formel 2,
X₂⁻ ein Halogenidion oder ein Ion einer organischen Säure darstellt; und
R₂ ein Wasserstoffatom oder eine Methylgruppe darstellt.

3. Verfahren zur Herstellung eines medizinischen Instruments nach Anspruch 1 oder 2, wobei das reaktive Monomer, das eine Epoxygruppe aufweist, mindestens eines umfasst, das ausgewählt ist aus der Gruppe, bestehend aus Glycidylacrylat, Glycidylmethacrylat, 3,4-Epoxycyclohexylmethylacrylat, 3,4-Epoxycyclohexylmethylmethacrylat, β-Methylglycidylmethacrylat und Allylglycidylether.

4. Verfahren zur Herstellung eines medizinischen Instruments nach einem der Ansprüche 1 bis 3, wobei das hydrophile Monomer mindestens eines umfasst, das ausgewählt ist aus der Gruppe, bestehend aus N,N-Dimethylacrylamid, Acrylamid, 2-Hydroxyethylmethacrylat und N-Vinylpyrrolidon.

5. Verfahren zur Herstellung eines medizinischen Instruments nach einem der Ansprüche 1 bis 4, wobei die Lösung 1 Massenprozent bis 10 Massenprozent des Blockcopolymers enthält.

6. Verfahren zur Herstellung eines medizinischen Instruments nach einem der Ansprüche 1 bis 5, wobei die Lösung das Blockcopolymer und das Cholin-Derivat in einem Massenverhältnis von 1 : 0,005 bis 1 : 1 enthält.

7. Medizinisches Instrument, das eine Basisschicht und
eine Gleitschicht umfasst, die auf mindestens einem Abschnitt der Basisschicht weitergeführt wird,
wobei die Gleitschicht ein Blockcopolymer, das eine Struktureinheit (A) aufweist, die von einem reaktiven Monomer mit einer Epoxygruppe abgeleitet ist, und eine Struktureinheit (B), die von einem hydrophilen Monomer abgeleitet ist, und ein Cholin-Derivat umfasst, wobei:
das Molverhältnis zwischen der Struktureinheit (A) und der Struktureinheit (B) des Blockcopolymers in einem Bereich von 1:10 bis 1:30 liegt, und
das Cholin-Derivat mindestens eines ist, das aus der Gruppe ausgewählt ist, die aus einer Verbindung der folgenden Formel 1 und einem Polymer besteht, das eine Struktureinheit aufweist, die durch die folgende Formel 2 dargestellt wird:
[Chem 5]
Formel 1 **R₁-O-(CH₂)₂-N⁺(CH₃)₃ X₁⁻**
wobei in Formel 1,
X₁⁻ ein Halogenidion, ein Ion einer organischen Säure oder ein Ion einer anorganischen Säure darstellt; und
R₁ ein Wasserstoffatom, eine substituierte oder unsubstituierte Alkylgruppe mit 1 bis 12 Kohlenstoffatomen oder Rₐ-C(=O)- darstellt, wobei Rₐ eine substituierte oder unsubstituierte Alkylgruppe, die 1 bis 12 Kohlenstoffatome aufweist, eine substituierte oder unsubstituierte Arylgruppe, die 6 bis 12 Kohlenstoffatome aufweist, CH₂=CH-, oder CH₂=C(CH₃)- darstellt; 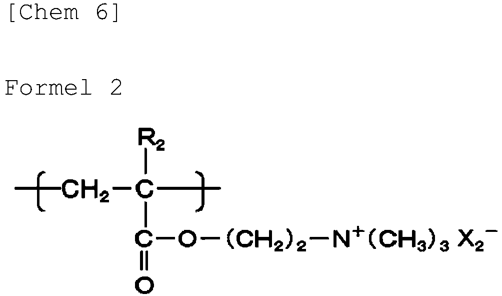
wobei in Formel 2,
X₂⁻ ein Halogenidion, ein Ion einer organischen Säure oder ein Ion einer anorganischen Säure darstellt; und
R₂ ein Wasserstoffatom oder eine Methylgruppe darstellt.

8. Medizinisches Instrument nach Anspruch 7, wobei die Gleitschicht das Blockcopolymer und das Cholin-Derivat in einem Massenverhältnis von 1 : 0,005 bis 1 : 1 enthält.

## Revendications

1. Procédé de fabrication d'un instrument médical comprenant une couche de base et une couche lubrifiante supportée par au moins une partie de la couche de base, le procédé comprenant :
l'application sur la couche de base d'une solution comprenant : un copolymère séquencé ayant une unité structurale (A) dérivée d'un monomère réactif ayant un groupe époxy et une unité structurale (B) dérivée d'un monomère hydrophile, un dérivé de choline et un solvant,
le rapport molaire entre l'unité structurale (A) et l'unité structurale (B) du copolymère séquencé étant compris entre 1:10 et 1:30, et
le dérivé de choline étant au moins un dérivé choisi dans le groupe constitué d'un composé représenté par la formule 1 suivante et d'un polymère ayant une unité structurale représentée par la formule 2 suivante :
[Chem 1]
Formule 1 **R₁-O-(CH₂)₂-N⁺(CH₃)₃ X₁⁻**
sachant que, dans la formule 1,
X₁⁻ représente un ion halogénure, un ion acide organique ou un ion acide inorganique : et
R₁ représente un atome d'hydrogène, un groupe alkyle substitué ou non substitué ayant 1 à 12 atomes de carbone, ou Rₐ-C(=O)-, Rₐ représente un groupe alkyle substitué ou non substitué ayant 1 à 12 atomes de carbone, un groupe aryle substitué ou non substitué ayant 6 à 12 atomes de carbone, CH₂=CH- ou CH₂=C(CH₃)- ; 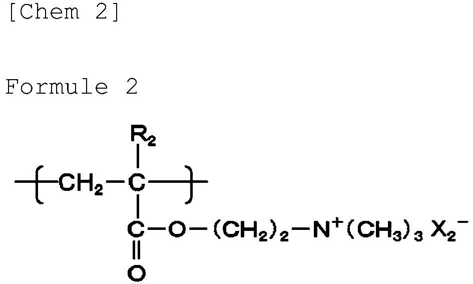
sachant que, dans la formule 2,
X₂⁻ représente un ion halogénure, un ion acide organique ou un ion acide inorganique : et
R₂ représente un atome d'hydrogène ou un groupe méthyle.

2. Procédé de fabrication d'un instrument médical selon la revendication 1, dans lequel le dérivé de choline est au moins un dérivé choisi dans le groupe constitué par un composé représenté par la formule 1 suivante et un polymère ayant une unité structurale représentée par la formule 2 suivante :
[Chem 3]
Formule 1 **R₁-O-(CH₂)₂-N⁺(CH₃)₃ X₁⁻**
sachant que, dans la formule 1,
X₁⁻ représente un ion halogénure ou un ion acide organique : et
R₁ représente un atome d'hydrogène ou Rₐ-C(=O)-, Rₐ représente un groupe alkyle substitué ou non substitué ayant 1 à 12 atomes de carbone, un groupe aryle substitué ou non substitué ayant 6 à 12 atomes de carbone, CH₂=CH- ou CH₂=C(CH₃)- ; 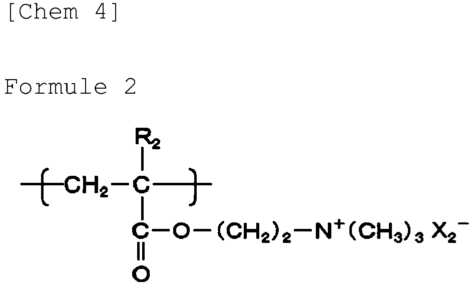
sachant que, dans la formule 2,
X₂⁻ représente un ion halogénure ou un ion acide organique : et
R₂ représente un atome d'hydrogène ou un groupe méthyle.

3. Procédé de fabrication d'un instrument médical selon la revendication 1 ou 2, dans lequel le monomère réactif ayant un groupe époxy comprend au moins un composé choisi dans le groupe constitué par l'acrylate de glycidyle, le méthacrylate de glycidyle, l'acrylate de 3,4-époxycyclohexylméthyle, le méthacrylate de 3,4-époxycyclohexylméthyle, le méthacrylate de β-méthylglycidyle, et l'éther allylglycidylique.

4. Procédé de fabrication d'un instrument médical selon l'une quelconque des revendications 1 à 3, dans lequel le monomère hydrophile comprend au moins un monomère choisi dans le groupe constitué par le N,N-diméthylacrylamide, l'acrylamide, le méthacrylate de 2-hydroxyéthyle et la N-vinylpyrrolidone.

5. Procédé de fabrication d'un instrument médical selon l'une quelconque des revendications 1 à 4, dans lequel la solution comprend 1 % à 10 % en masse du copolymère séquencé.

6. Procédé de fabrication d'un instrument médical selon l'une quelconque des revendications 1 à 5, dans lequel la solution comprend le copolymère séquencé et le dérivé de choline avec un rapport massique de 1 : 0,005 à 1 : 1.

7. Instrument médical comprenant une couche de base et une couche lubrifiante supportée par au moins une partie de la couche de base,
la couche lubrifiante comprenant un copolymère séquencé ayant une unité structurale (A) dérivée d'un monomère réactif ayant un groupe époxy et une unité structurale (B) dérivée d'un monomère hydrophile, et un dérivé de choline,
le rapport molaire entre l'unité structurale (A) et
l'unité structurale (B) du copolymère séquencé étant compris entre 1:10 et 1:30, et
le dérivé de choline étant au moins un dérivé choisi dans le groupe constitué d'un composé représenté par la formule 1 suivante et d'un polymère ayant une unité structurale représentée par la formule 2 suivante :
[Chem 5]
Formule 1 **R₁-O-(CH₂)₂-N⁺(CH₃)₃ X₁⁻**
sachant que, dans la formule 1,
X₁⁻ représente un ion halogénure, un ion acide organique ou un ion acide inorganique : et
R₁ représente un atome d'hydrogène, un groupe alkyle substitué ou non substitué ayant 1 à 12 atomes de carbone, ou Rₐ-C(=O)-, Rₐ représente un groupe alkyle substitué ou non substitué ayant 1 à 12 atomes de carbone, un groupe aryle substitué ou non substitué ayant 6 à 12 atomes de carbone, CH₂=CH- ou CH₂=C(CH₃)- ; 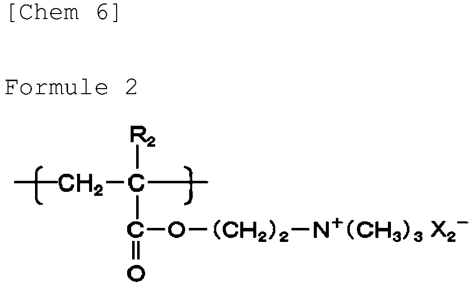
sachant que, dans la formule 2,
X₂⁻ représente un ion halogénure, un ion acide organique ou un ion acide inorganique : et
R₂ représente un atome d'hydrogène ou un groupe méthyle.

8. Instrument médical selon la revendication 7, dans lequel la couche lubrifiante comprend le copolymère séquencé et le dérivé de choline selon un rapport massique de 1 : 0,005 à 1 : 1.
